# EUROPEAN PATENT SPECIFICATION

(11) **EP 4 493 269 B1**
(45) Date of publication and mention of the grant of the patent: **27.05.2026**
(21) Application number: 23712089.4
(22) Date of filing: 06.03.2023
(51) Int. Cl.: A61N 1/362, A61N 1/37, A61N 1/39, A61N 1/372

(54) **MEDICAL DEVICE WITH DETECTION OF AN INTEGRATED OR TRUE BIPOLAR LEAD FOR SELECTING OPERATING PARAMETERS**
MEDIZINISCHE VORRICHTUNG MIT DETEKTION EINER INTEGRIERTEN ODER WAHREN BIPOLAREN LEITUNG ZUR AUSWAHL VON BETRIEBSPARAMETERN
DISPOSITIF MÉDICAL AVEC DÉTECTION D'UN CONDUCTEUR BIPOLAIRE INTÉGRÉ OU VRAI POUR SÉLECTIONNER DES PARAMÈTRES DE FONCTIONNEMENT

(30) Priority: 16.03.2022 US 202263320611 P
(43) Date of publication of application: 22.01.2025
(73) Proprietor: Medtronic, Inc., Minneapolis, Minnesota 55432 (US)
(72) Inventor: SCHILLING, Eric A., Minneapolis, Minnesota 55432 (US); BOUNDS, Chad A., Minneapolis, Minnesota 55432 (US)
(74) Representative: Zimmermann & Partner Patentanwälte mbB
(86) International application number: PCT/IB2023/052080
(87) International publication number: WO 2023/175440

(56) References cited:
- WO-A1-2005/056109
- US-A1- 2004 064 161
- US-A1- 2006 116 733
- US-A1- 2007 233 217
- US-A1- 2008 161 873
- US-A1- 2009 326 600
- US-A1- 2017 128 732
- US-B2- 10 434 315

## Description

### TECHNICAL FIELD

The disclosure relates generally to a medical device for selecting one or more operating parameters of the medical device based on detecting connection of either an integrated bipolar lead or a true bipolar lead to the medical device.

### BACKGROUND

Medical devices may sense electrophysiological signals from the heart, brain, nerve, muscle or other tissue. Such devices may be implantable, wearable or external devices using implantable and/or surface (skin) electrodes for sensing the electrophysiological signals. In some cases, such devices may be configured to deliver a therapy based on the sensed electrophysiological signals. For example, implantable or external cardiac pacemakers, cardioverter defibrillators, cardiac monitors and the like, sense cardiac electrical signals from a patient's heart. The medical device may sense cardiac electrical signals from the heart and deliver electrical stimulation therapies, such as cardiac pacing pulses and/or cardioversion or defibrillation (CV/DF) shocks, to the heart using electrodes, which may be carried by medical electrical leads extending from the medical device to position electrodes within or near the patient's heart.

Cardiac signals sensed from the heart may be analyzed for detecting an abnormal rhythm. Upon detection of an abnormal rhythm, such as bradycardia, tachycardia or fibrillation, an appropriate electrical stimulation pulse or pulses may be delivered to restore or maintain a more normal rhythm of the heart. For example, an implantable cardioverter defibrillator (ICD) may deliver bradycardia pacing pulses to the heart of the patient in the absence of sensed intrinsic myocardial depolarization signals, e.g., R-waves, deliver anti-tachycardia pacing (ATP) pulses in response to detecting tachycardia, or deliver CV/DF shocks to the heart upon detecting tachycardia or fibrillation.

A variety of medical electrical leads and associated electrodes have been proposed or are commercially available for use with medical device for sensing electrophysiological signals and may also be used for delivering electrical stimulation pulses in some clinical applications. Medical electrical leads may be manufactured with industry standard connector assemblies for connecting the lead to a medical device. For example, a DF-4 connector is an industry standard medical lead connector that connects a medical lead having at least one high surface area electrode to an ICD for delivering CV/DF shocks. Medical leads having an industry standard connector may be connected to various medical devices that may be available from different manufacturers. Such medical leads having an industry standard connector, however, may have electrode configurations that differ from one another even though the lead connector conforms with an industry standard.Document US 2006/116733 A1 relates to a method and apparatus for identifying lead-related
conditions using prediction and detection criteria. Document US 2008/161873 A1 relates to a method and apparatus for reducing inappropriate detection of lead-related noise.
Document WO 2005/056109 A1 relates to a method and apparatus for identifying lead-related conditions using impedance trends and oversensing criteria.
Document US 2004/064161 A1 relates to a method and apparatus for identifying lead-related conditions using lead impedance measurements.
Document US 2017/128732 A1 relates to connectivity detection and type identification of an implanted lead for an implantable medical device.
Document US 10,434,315 B2 relates to system and methods for automatically determining pace and sense configurations for an implantable device.

### SUMMARY

In general, the disclosure is directed to an implantable medical device (IMD) and techniques for detecting connection of a true bipolar lead or an integrated bipolar lead to the IMD. The IMD is configured to select at least one operational parameter based on the type of lead detected. The type of lead may be detected based on an impedance measurement performed between a pair of electrode terminals. The IMD includes signal processing circuitry that can be configured to receive a cardiac electrical signal via the medical lead and process the cardiac electrical signal according to at least one operating parameter set based on the detected lead type. The cardiac electrical signal may be processed for determining a need for an electrical stimulation therapy in some examples. The IMD may be configured to deliver the electrical stimulation therapy in response to determining the need for the electrical stimulation therapy.

In one example, the disclosure provides a medical device including a connector bore configured to receive a proximal portion of a medical lead. The connector bore includes at least a first electrical contact and a second electrical contact. The medical device further includes an impedance measurement circuit configured to obtain an impedance measurement between a first electrode terminal corresponding to the first electrical contact of the connector bore and a second electrode terminal corresponding to the second electrical contact of the connector bore. The medical device further includes a control circuit configured to determine a medical lead type of the medical lead received by the connector bore based on the impedance measurement. The medical lead type is determined to be one of an integrated bipolar lead or a true bipolar lead. The control circuit can be further configured to select at least one operating parameter setting based on the determined medical lead type. The medical device further includes signal processing circuitry configured to receive a cardiac electrical signal and process the cardiac electrical signal according to the at least one operating parameter setting for determining a need for an electrical stimulation therapy. The medical device may further include a therapy delivery circuit configured to deliver the electrical stimulation therapy in response to the signal processing circuitry determining the need for the electrical stimulation therapy.

In another example, the disclosure provides a method that includes obtaining, by an impedance measurement circuit of a medical device, an impedance measurement between a first electrode terminal corresponding to a first electrical contact of a connector bore of the medical device and a second electrode terminal corresponding to a second electrical contact of the connector bore. The connector bore is configured to receive a proximal portion of a medical lead. The method further includes determining, by a control circuit of the medical device, a medical lead type of the medical lead received by the connector bore based on the impedance measurement, wherein the medical lead type is determined to be one of an integrated bipolar lead or a true bipolar lead. The method further includes selecting at least one operating parameter setting based on the determined medical lead type, processing by signal processing circuitry of the medical device a cardiac electrical signal according to the at least one operating parameter setting for determining a need for an electrical stimulation therapy, and delivering the electrical stimulation therapy by a therapy delivery circuit of the medical device in response to determining the need for the electrical stimulation therapy.

In yet another example, the disclosure provides a non-transitory computer readable medium storing a set of instructions that, when executed by a control circuit of a medical device, cause the medical device to obtain an impedance measurement between a first electrode terminal corresponding to a first electrical contact of a connector bore of the medical device and a second electrode terminal corresponding to a second electrical contact of the connector bore. The connector bore being configured to receive a proximal portion of a medical lead. The instructions further cause the medical device to, based on the impedance measurement, determine a medical lead type of the medical lead received by the connector bore as being one of an integrated bipolar lead or a true bipolar lead and select at least one operating parameter setting based on the determined medical lead type. The instructions further cause the medical device to process a cardiac electrical signal according to the at least one operating parameter setting for determining a need for an electrical stimulation therapy and deliver the electrical stimulation therapy in response to determining the need for the electrical stimulation therapy. Further aspects, embodiments and examples disclosed herein which do not fall under the scope of the appended claims do not form part of the claimed aspect of the invention and are merely provided for illustrative purposes.

This summary is intended to provide an overview of the subject matter described in this disclosure. It is not intended to provide an exclusive or exhaustive explanation of the apparatus described in detail within the accompanying drawings and description below. Further details of one or more examples are set forth in the accompanying drawings and the description below and from the claims.

### BRIEF DESCRIPTION OF DRAWINGS

FIG. 1 is a conceptual diagram of a medical device system configured to sense cardiac electrical signals and deliver electrical stimulation therapies to a patient's heart according to some examples.
FIG. 2 is a conceptual diagram of a sectional view of an IMD connector block and a proximal lead assembly of a true bipolar lead according to some examples.
FIG. 3 is a conceptual diagram of the IMD of FIG. 1 connected to a lead and electrode configuration according to another example.
FIG. 4 is a conceptual diagram of proximal lead connector assembly of an integrated bipolar lead inserted into an IMD connector block according to another example.
FIG. 5 is a conceptual diagram of the IMD of FIGs. 1 and 3 according to some examples.
FIG. 6 is a flow chart of a method that may be performed by an IMD for determining a connected lead type as being either a true bipolar lead or an integrated bipolar lead and selecting an operating parameter based on the connected lead type.
FIG. 7 is a flow chart of a method that an IMD may perform for selecting operating and report parameters in response to detecting an integrated bipolar lead connected to the IMD.
FIG. 8 is a diagram of a graphical user interface (GUI) that may be displayed by the external device of FIG. 1 in response to receiving data transmitted from an IMD according to one example.
FIG. 9 is a diagram of a data collection setup GUI that may be displayed by the external device of FIG. 1 according to another example.
FIG. 10 is a diagram of a GUI that may include data and information based on a detected lead type according to another example.
FIG. 11 is another example of a GUI that includes a report of lead impedance measurements that may be reported according to a detected lead type according to another example.

### DETAILED DESCRIPTION

In general, this disclosure describes medical devices and techniques for selecting operating and/or reporting parameters based on the type of medical electrical lead connected to the medical device. An IMD, e.g., a cardiac pacemaker or ICD, can be configured to sense electrical activity of the heart, e.g., by sensing cardiac event signals attendant to the electrical depolarizations occurring in one or more heart chambers of a patient. A cardiac electrical signal can be received by the IMD in a raw form from electrodes placed in, on or in operative proximity to the heart by one or more lead(s) for processing and analysis by the IMD. The cardiac electrical signals can be processed and analyzed by signal processing circuitry of the IMD for detecting abnormal heart rhythms. For instance, signal processing circuitry of the IMD can be configured to receive a cardiac electrical signal via a medical lead coupled to the IMD and process the cardiac electrical signal according to various operating parameters for determining a need for an electrical stimulation therapy. Using the techniques disclosed herein, one or more operating parameters used by the signal processing circuitry for determining a heart rhythm or a need for electrical stimulation therapy may be set by control circuitry of the IMD based on the type of medical electrical lead connected to the IMD. The IMD may deliver an electrical stimulation therapy, e.g., a cardiac pacing therapy or a CV/DF shock via electrodes coupled to the IMD when a need for electrical stimulation therapy is determined.

The IMD may acquire and store data relating to a detected heart rhythm and/or control electrical stimulation therapies delivered to the heart in response to detecting an abnormal rhythm. A variety of cardiac lead and electrode configurations are available such that different leads, which may carry a different number and/or types of electrodes, may be coupled to a given IMD. Operating parameters of the IMD and/or diagnostic information acquired by the IMD and transmitted to an external device or computer for display on a graphical user interface (GUI) to a clinician or other user may be different for one type of lead and electrode configuration than for another type of lead and electrode configuration. The techniques disclosed herein provide for detecting the type of lead and electrodes coupled to an IMD and automatic selection of one or more operating parameters used in determining a need for electrical stimulation therapy and/or reporting data acquired by the IMD based on the type of lead and electrodes detected as being connected to the IMD.

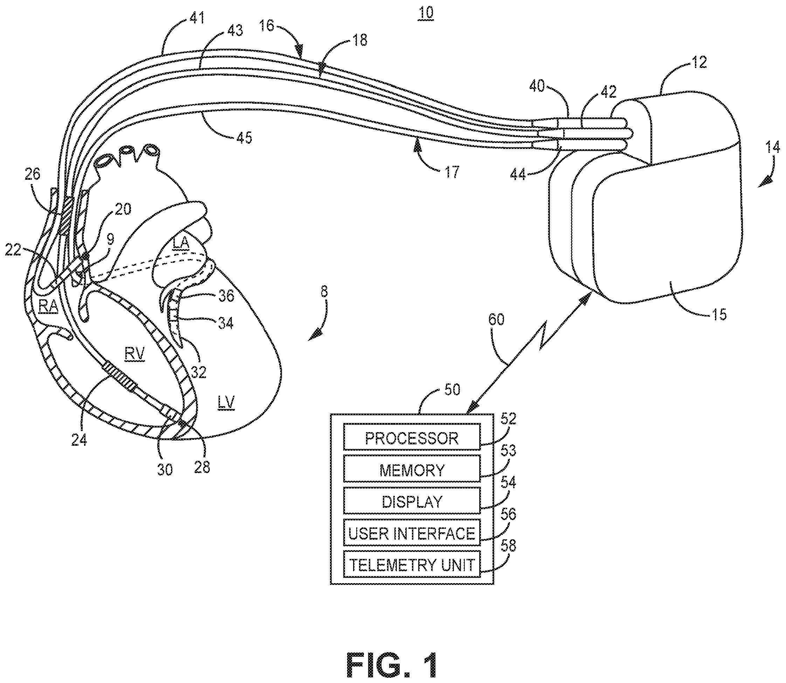

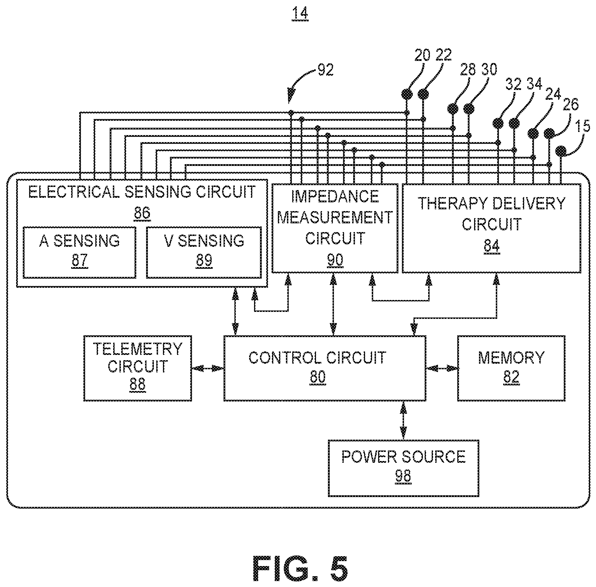

FIG. 1 is a conceptual diagram of a medical device system 10 configured to sense cardiac electrical signals and deliver electrical stimulation therapies to a patient's heart 8. Medical device system 10 includes IMD 14 coupled to a patient's heart 8 via transvenous electrical leads 16, 17 and 18. IMD 14 is shown as a multi-chamber device capable of delivering cardiac pacing pulses and sensing cardiac electrical signals in the right atrium (RA), the right ventricle (RV) and the left ventricle (LV). IMD housing 15 encloses internal circuitry corresponding to the various circuits and components described in conjunction with FIG. 5 below, for performing the functionality of IMD 14 as disclosed herein, including performing lead impedance measurements, determining a type of medical lead coupled to IMD 14, selecting one or more operating parameters based on the determined medical lead type, sensing cardiac signals from heart 8, detecting arrhythmias, storing and transmitting data and controlling therapy delivery.

IMD housing 15 forms a hermetic seal that protects internal components of IMD 14. Housing 15 may be formed of a conductive material, such as titanium or titanium alloy. Housing 15 may function as an electrode (sometimes referred to as a "can" electrode). Housing 15 may be used as an active can electrode for use in delivering high voltage CV/DF shock pulses to heart 8 for terminating a tachyarrhythmia, e.g., ventricular tachycardia or fibrillation. In other examples, housing 15 may be available for use in delivering unipolar, relatively lower voltage cardiac pacing pulses and/or for sensing cardiac electrical signals in combination with electrodes carried by a lead coupled to IMD 14. In other instances, the housing 15 of IMD 14 may include a multiple electrodes on an outer portion of the housing. The outer portion(s) of the housing 15 functioning as an electrode(s) may be coated with a material, such as titanium nitride, e.g., for reducing post-stimulation polarization artifact.

IMD 14 includes a connector block 12 (sometimes called a "header") that includes insulated electrical feedthroughs crossing housing 15 to provide electrical connections between conductors extending within the leads 16, 17 and 18 coupled to IMD 14 and the electronic components enclosed by housing 15. As described below in conjunction with FIG. 5, housing 15 may enclose one or more processing circuits, memories, transceivers, cardiac electrical signal sensing circuitry, therapy delivery circuitry, power sources and other components for sensing cardiac electrical signals, processing and analyzing sensed cardiac electrical signals, and delivering electrical stimulation pulses as needed.

In the example shown, connector block 12 is configured to receive a proximal lead connector assembly 40, 42 and 44 of each of RA lead 16, RV lead 18 and LV lead 17, respectively. Each lead 16, 17 and 18 can be advanced transvenously for positioning electrodes for sensing and stimulation in the atria or ventricles of heart 8. The proximal portion of each of lead 16, 18 and 17 may be configured as an industry standard or custom lead connector assembly 40, 42 and 44, respectively. Connector block 12 includes connector bores that are appropriately sized for receiving the proximal portion of each lead 16, 18 and 17, e.g., lead connector assemblies 40, 42 or 44. Each connector bore includes electrical contacts that become aligned with and physically mate with a corresponding electrical contact of the respective lead connector assembly 40, 42 or 44 providing physical and electrical connection of each lead 16, 18 and 17 to IMD 14.

RA lead 16 includes an elongated lead body 41, a proximal lead connector assembly 40 and distal electrodes 20 and 22 in the example shown. RA lead 16 may be advanced transvenously for positioning its distal end, carrying electrodes 20 and 22, in the vicinity of the RA. RA lead 16 is equipped with pacing and sensing electrodes 20 and 22, shown as a tip electrode 20 and a ring electrode 22 spaced proximally from tip electrode 20 along RA lead body 41. Tip electrode 20 may be used as a cathode electrode with ring electrode 22 serving as an anode electrode for bipolar pacing and bipolar sensing in the RA. The electrodes 20 and 22 are each connected to a respective insulated conductor extending within the elongated body 41. Each insulated conductor is coupled at its proximal end to an electrical connector (not shown in FIG. 1), e.g., a pin or ring connector, of the proximal lead connector assembly 40.

RV lead 18 includes an elongated lead body 43 having a proximal connector assembly 42 at its proximal end for coupling lead 43 to IMD connector block 12 and electrodes 24, 26, 28 and 30 carried along a distal portion of lead body 43. RV lead 18 may be advanced transvenously through the RA and into the RV to position electrodes 24, 28 and 30 in the RV. RV lead 18 is shown carrying a distal tip electrode 28 and ring electrode 30 spaced proximally from tip electrode 28 for bipolar sensing of cardiac electrical signals in the RV and delivering bipolar cardiac pacing pulses. Tip electrode 28 may be used as a cathode electrode for pacing and sensing with ring electrode 30 serving as an anode electrode.

RV lead 18 is further shown to be carrying an RV coil electrode 24 spaced proximally from ring electrode 30 and a superior vena cava (SVC) coil electrode 26 spaced proximally from RV coil electrode 24. SVC coil electrode 26 may be carried along RV lead body 43 such that it is positioned at least partially within the RA and/or SVC when the distal end of RV lead 18 is advanced within the right ventricle. Coil electrodes 24 and 26 are elongated electrodes having a relatively high surface area compared to electrodes 20, 22, 28 and 30. Coil electrodes 24 and 26 may have a surface area ranging from 50 to 100 times greater than the surface area of electrodes 20, 22, 28 and 30, for example. For the sake of convenience, electrodes 24 and 26 are referred to herein as "coil electrodes" because they may take the form of a coiled electrode, which may include a single wire or filar or multiple wires or filars (e.g., a braided multi-filar wire, a stranded multi-filar wire, etc.) that winds helically around a longitudinal portion of lead body 43 to provide a relatively high surface area electrode for delivering high voltage CV/DF shocks. However, it is to be understood that electrodes 24 and 26 may be configured as other types of high surface area electrodes that can be used for delivering CV/DF shocks, which may include ribbon electrodes, plate electrodes, serpentine electrodes, zig-zagging electrodes, segmented electrodes or other types of physical electrode configurations that provide a relatively large surface area and low impedance that do not necessarily include a coiled wire.

Coil electrodes 24 and 26 (and in some examples housing 15) are sometimes referred to as "defibrillation electrodes" or "CV/DF electrodes" because they can be utilized, individually or collectively, for delivering high voltage CV/DF shocks. However, in some examples, as described below, a coil electrode available for delivering CV/DF shocks may be utilized in a cardiac sensing electrode vector to sense cardiac electrical signals. In this sense, the use of the term "defibrillation electrode" or "CV/DF electrode" herein should not be considered as limiting the coil electrodes 24 and 26 for use in only high voltage CV/DF shock therapy applications. For example, either of coil electrodes 24 and 26 may be used as a sensing electrode in a sensing electrode vector for sensing cardiac electrical signals and determining a need for an electrical stimulation therapy. While two coil electrodes 24 and 26 are shown along lead body 43 of RV lead 18, in other examples only one coil electrode, e.g., RV coil electrode 24 (which may be used in combination with housing 15 for delivering high voltage shock pulses), or more than two coil electrodes may be carried by lead body 43. In still other examples, two or more coil electrodes may be carried by two or more different lead bodies extending from IMD 14. For example, in some lead and electrode configurations, SVC coil electrode 26 may be carried by lead body 41 of RA lead 16. Housing 15 may function as an active electrode during CV/DF shock delivery in conjunction with RV coil electrode 24 and/or SVC coil electrode 26 in some examples.

Each of electrodes 24, 26, 28 and 30 carried by RV lead body 43 are connected to a respective insulated conductor extending within lead body 43 of RV lead 17. Lead body 43 may be a multi-lumen lead body in some examples to accommodate multiple, insulated conductors. The proximal ends of the insulated conductors are coupled to corresponding electrical connectors (not illustrated in FIG. 1) of proximal lead connector assembly 42 for providing electrical connection to IMD 14.

The RV lead tip electrode 28 and the RA lead tip electrode 20 can be active fixation electrodes providing fixation of the distal ends of leads 18 and 16, respectively, at an implant site in addition to providing cardiac electrical signal sensing and cardiac pacing functionality. In FIG. 1, RA tip electrode 20 and RV tip electrode 28 are each shown as a helical, screw-in electrode that can be rotatably advanced into cardiac tissue to provide lead fixation. In other examples, tip electrodes 20 and 28 may be configured as fishhook electrodes, hemispherical electrodes, button electrodes or other types of electrodes. When the tip electrode of the medical lead does not provide fixation of the distal end of the elongated lead body, the RA lead 16 or RV lead 18 may be equipped with other fixation mechanisms, such as tines or hooks, that may engage with cardiac tissue at an implant site.

The proximal ring electrode 22 of RA lead 16 and the proximal ring electrode 30 of RV lead 18 may each be ring electrodes that fully or partially circumscribe the respective lead body 41 or 43. In various examples, the relatively low surface area pace/sense electrodes 20, 22, 28 and 30 may be implemented as ring electrodes, short coil electrodes, button electrodes, hemispherical electrodes, directional electrodes, segmented electrodes, helical electrodes, fishhook electrodes, or other shaped electrode and are not limited to being exclusively ring electrodes and helical screw-in electrodes as shown here.

RA lead electrodes 20 and 22 and RV lead electrodes 28 and 30 are relatively small surface area electrodes which are available for use in sensing cardiac electrical signals and may be used in for delivering relatively low voltage cardiac pacing pulses, e.g., for delivering bradycardia pacing, post-shock pacing, cardiac resynchronization therapy (CRT), ATP therapy or other therapeutic cardiac pacing pulses. In some cases, RV lead electrodes 28 and 30 may be used to deliver high frequency induction pulses delivered to induce a tachyarrhythmia, e.g., during CV/DF threshold testing. Electrodes 20, 22, 28 and 30 are sometimes referred to as "pace/sense electrodes" because they are generally configured for use in low voltage applications, e.g., used as either a cathode or anode for delivery of pacing pulses and/or sensing of cardiac electrical signals, as opposed to delivering high voltage CV/DF shocks. In some instances, electrodes 20, 22, 28 and 30 may provide only pacing functionality, only sensing functionality or both.

LV lead 17 includes elongated lead body 45 having a proximal connector assembly 44 and distal electrodes 34 and 36, which may be provided as any of the example pacing and sensing electrodes listed above. LV lead 17 may be advanced transvenously into the RA, and further into a cardiac vein 32 via the ostium of the coronary sinus 9 to position electrodes 34 and 36 along the lateral free wall of the left ventricle. Electrodes 34 and 36 may be used as a bipolar sensing and pacing electrode pair for sensing cardiac electrical signals from the LV and for delivering LV pacing pulses. LV lead 17 may be coupled to IMD 14 for providing multi-chamber sensing and therapy delivery to heart 8, such as CRT.

CRT may be delivered when poor ventricular synchrony between the RV and the LV exists, e.g., due to conduction disease, heart failure or other cardiac conditions. LV lead 17 may be optional in some examples and may not be included in some medical device systems that employ the techniques disclosed herein. While LV lead 17 is shown as a bipolar lead having two electrodes 34 and 36, LV lead 17 may be a unipolar lead having one electrode or a multi-polar lead, e.g., having three or four electrodes. In some examples, LV lead 17 is a quadripolar lead having four electrodes, e.g., four ring electrodes or one tip electrode and three ring electrodes, with proximal connector assembly 44 configured as an industry standard IS-4 connector.

Electrodes 20, 22, 24, 26, 28, 30, 34 and 36 may be formed from titanium, platinum, iridium or alloys thereof, as examples with no limitation intended, and may include a low polarizing coating, such as titanium nitride, iridium oxide, ruthenium oxide, platinum black, among others. Lead bodies 41, 43 and 45 may each be formed from a nonconductive material, including silicone, polyurethane, fluoropolymers, mixtures thereof, and/or other appropriate materials. Each lead body may be shaped to form one or more lumens within which one or more insulated electrical conductors extend between the electrical connectors of the proximal lead connectors 40, 42 and 44 and the respective electrodes carried by the lead body. The lead bodies 41, 43, and 45 may be generally tubular or cylindrical in shape but may have a flattened or ribbon shape in some examples. Any of the lead bodies 41, 43 and 45 may have a pre-formed shape such as a curve or bend, which may be along a distal portion of the lead body, to facilitate guidance and implantation of the lead body distal end at a targeted implant site. In other examples, the lead bodies 41, 43 and 45 may be elongated flexible bodies without any preformed shapes or curves.

In the example shown in FIG. 1, RA lead 16 and RV lead 18 are configured as "true bipolar" leads in that a cardiac electrical signal can be sensed between tip electrode 20 and ring electrode 22 in the RA, and a cardiac electrical signal can be sensed between tip electrode 28 and ring electrode 30 in the RV. As used herein, a "true bipolar lead" refers to a lead having a pair of relatively small surface area electrodes that are carried at an inter-electrode distance along the lead body so that both of the electrodes are located within or along a single heart chamber for sensing relatively local cardiac electrical signals. A true bipolar lead, such as RV lead 18, may carrying other electrodes, such as CV/DF electrodes 24 and 26, that are coupled to electrical conductors extending within the lead body that are electrically isolated from the other electrodes and respective conductors of the lead. Referring to RV lead 18 as an example, ring electrode 30 is provided for bipolar sensing and pacing in the RV with tip electrode 28. Ring electrode 30 may have a dedicated conductor extending through lead body 43 that is electrically insulated from other conductors and electrodes of RV lead 18.

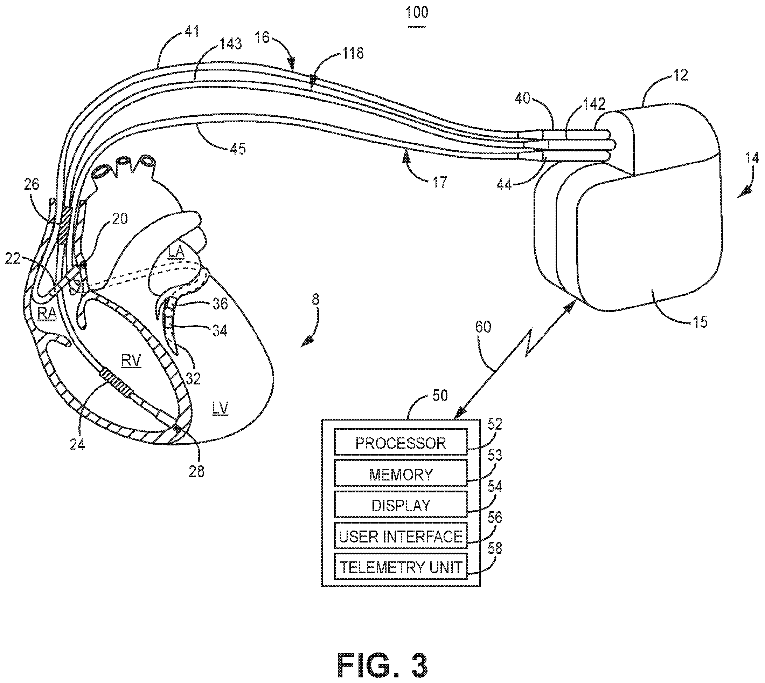

In contrast, an "integrated bipolar" lead refers to a lead that is configured to sense cardiac electrical signals using a tip electrode and a coil electrode, e.g., RV tip electrode 28 and RV coil electrode 24, omitting the need for a ring electrode. As described below in conjunction with FIG. 3, in an "integrated bipolar" lead, the RV coil electrode 24 may serve the dual purposes of bipolar sensing of cardiac electrical signals when paired with tip electrode 28 and delivering high voltage CV/DF shocks in combination with SVC coil electrode 26 and/or housing 15.

The inter-electrode distance of a bipolar pair of electrodes carried by a true bipolar lead, sometimes referred to as a "dedicated" bipolar lead, may be 10 to 20 millimeters (mm), or about 12 to 15 mm. A true bipolar sensing electrode vector between a tip electrode and a ring electrode spaced proximally from the tip electrode is sometimes referred to as a "tip-to-ring" sensing electrode vector. However, a true bipolar pair of electrodes could include two ring electrodes or other types of electrodes carried along the lead body for positioning within or along a targeted heart chamber. When a tip-to-ring electrode vector is used for bipolar sensing and pacing in a given heart chamber, the ring electrode may or may not make contact with the endocardium.

It is to be understood that although IMD 14 is described as a multi-chamber device capable of sensing and pacing in the RA, RV and LV, in other examples, IMD 14 may be a dual chamber device, e.g., coupled to RA lead 16 and RV lead 18. In still other examples, IMD 14 may be a single chamber device, e.g., coupled only to RA lead 16 or only to RV lead 18. IMD 14 is described as being an ICD capable of delivering both low voltage cardiac pacing therapies and high voltage CV/DF shocks, IMD 14 may be configured as a single, dual or multi-chamber pacing device without necessarily having CV/DF shock delivery capabilities.

An external device 50 is shown in telemetric communication with IMD 14 by a communication link 60. External device 50 may be embodied as a programmer used in a hospital, clinic or physician's office to retrieve data from IMD 14 and to program operating parameters and algorithms in IMD 14 for controlling IMD functions. External device 50 may alternatively be embodied as a home monitor or handheld device for retrieving data from IMD 14. External device 50 may be used to program cardiac signal sensing parameters, cardiac rhythm detection parameters, pacing and CV/DF therapy control parameters and other operating and control parameters used by IMD 14.

External device 50 may include a processor 52, memory 53, display unit 54, user interface 56 and telemetry unit 58. Processor 52 executes instructions stored in memory 53. Processor 52 may include any one or more of a microprocessor, a controller, a digital signal processor (DSP), an application specific integrated circuit (ASIC), a fieldprogrammable gate array (FPGA), or equivalent discrete or analog logic circuitry. In some examples, processor 52 may include multiple components, such as any combination of one or more microprocessors, one or more controllers, one or more DSPs, one or more ASICs, or one or more FPGAs, as well as other discrete or integrated logic circuitry. The functions attributed to processor 52 herein may be embodied as software, firmware, hardware or any combination thereof.

Memory 53 may include any volatile, non-volatile, magnetic, optical, or electrical media, such as a random-access memory (RAM), read-only memory (ROM), non-volatile RAM (NVRAM), electrically-erasable programmable ROM (EEPROM), flash memory, or any other digital or analog media. Memory 53 may be configured to store instructions executed by processor 52 for obtaining data received from IMD 14 and for generating a GUI on display unit 54 according to the techniques disclosed herein. Memory 53 may store various operating parameter settings of IMD 14 that may be used in generating various GUI windows, menus, reports, etc. by processor 52 based at least in part on a type of medical lead detected by IMD 14, as further described below.

Display unit 54 may generate a display of cardiac electrical signals, lead impedance measurements, programmed operating settings of IMD 14, operating settings selected based on a detected lead type and other device and patient related data received from processor 52. Display unit 54 may be configured to generate a GUI including various windows, icons, user selectable menus, etc. to facilitate interaction by a user with the external device 50. Display unit 54 may function as an input and/or output device using technologies including liquid crystal displays (LCD), quantum dot display, dot matrix displays, light emitting diode (LED) displays, organic light-emitting diode (OLED) displays, cathode ray tube displays, e-ink, or monochrome, color, or any other type of display capable of generating tactile, audio, and/or visual output. In some examples, display unit 54 is a presence-sensitive display that may serve as a user interface device that operates both as one or more input devices and one or more output devices.

User interface unit 56 may include a mouse, touch screen, keypad or the like to enable a user to interact with external device 50, e.g., to initiate and terminate an interrogation session for retrieving data from IMD 14, adjust settings of display unit 54, enter programming commands or selections or make other user requests. Telemetry unit 58 includes a transceiver and antenna configured for bidirectional communication with a telemetry circuit included in an IMD 14, e.g., in response to user requests.

Telemetry unit 58 is configured to operate in conjunction with processor 52 for sending and receiving data relating to IMD functions via a wireless communication link 60 with IMD 14. Communication link 60 may be established using a radio frequency (RF) link such as BLUETOOTH^{®}, Wi-Fi, Medical Implant Communication Service (MICS) or other communication bandwidth. In some examples, external device 50 may include a programming head that is placed proximate IMD 14 to establish and maintain a communication link 60, and in other examples external device 50 and IMD 14 may be configured to communicate using a distance telemetry algorithm and circuitry that does not require the use of a programming head and does not require user intervention to maintain a communication link.

It is contemplated that external device 50 may be in wired or wireless connection to a communications network via telemetry circuit 58 that includes a transceiver and antenna or via a hardwired communication line for transferring data to a centralized database or computer to allow remote management of the patient. Remote patient management systems including a centralized patient database may be configured to utilize the presently disclosed techniques to produce reports of data retrieved from IMD 14. One example of a remote patient management system that may be used in conjunction with the techniques disclosed herein is the CARELINK^{®} Network (Medtronic, Inc. Minneapolis, MN). Review of operating parameter settings and other data collected from IMD 14 may be performed remotely by a clinician who may authorize programming of operating parameters in IMD 14, e.g., after viewing reports and cardiac electrical signals and other device related data, such as marker channel data and therapy delivery history.

As described below, IMD 14 may determine a type of medical electrical lead connected to IMD 14 as being either a true bipolar lead or an integrated bipolar lead. IMD 14 may self-configure one or more operating parameters and transmit a lead type notification signal to external device 50. External device 50 may be configured to generate a GUI on display unit 54, which may include IMD operating parameters that are userprogrammable to settings selected based on the determined medical lead type and IMD operating parameters that may be selected by IMD 14 based on the determined medical lead type that may not be user programmable or adjustable. In some examples a recommended setting may be displayed in the GUI based on the detected medical lead type, and the user may be able to program the operating parameter to the recommended setting or another setting. Such programming operations, or approval of recommended programming settings, may be performed remotely from the physical location of the patient via a remote patient management system in communication with external device 50.

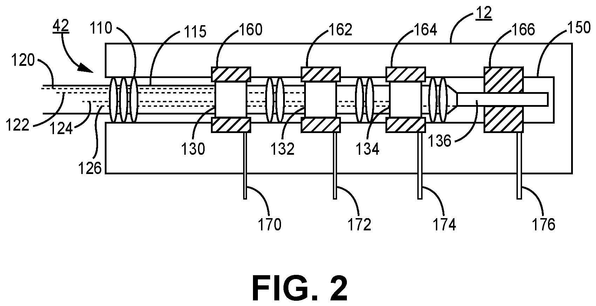

FIG. 2 is a conceptual diagram of a sectional view of connector block 12 and proximal lead assembly 42 of true bipolar lead 18 shown in FIG. 1. Connector block 12 is shown having single connector bore 150 for receiving the proximal portion of RV lead 16, i.e., lead connector assembly 42, for the sake of clarity. However, it is understood that when IMD 14 is a dual chamber or multi-chamber pacemaker or ICD, connector block 12 may include two or three connector bores, respectively, to receive each of an RA lead, RV lead and LV lead as generally shown in FIG. 1.

Lead connector assembly 42 includes an assembly body 115 carrying electrical ring connectors 130, 132, 134 and pin connector 136, each electrically coupled to a respective insulated electrical conductor 120, 122, 124 and 126 that extends through RV lead body 43 (shown in FIG. 1) to a respective distal electrode, e.g., SVC coil electrode 26, RV coil electrode 24, RV ring electrode 30 and RV tip electrode 28 as shown in FIG. 1. Lead connector assembly 42 may include one or more sealing rings 110 positioned distally from each of the connectors 130, 132, 134 and 136 to minimize intrusion of body fluids into connector block bore 150 and to provide electrical insulation between connectors 130, 132, 134 and 136.

Connector block bore 150 has an inner diameter sized to receive lead connector assembly 42. Connector block bore 150 includes multiple electrical contacts 160, 162, 164 and 166 distributed along bore 150. When lead connector assembly 42 is fully inserted into connector bore 150, each of the electrical connectors 130, 132, 134 and 136 of lead connector assembly 42 becomes aligned with and in electrical contact with a respective connector bore contact 160, 162, 164 and 166. In some examples, connector block 12 may include set screws that can be tightened down onto lead connector assembly 42 to reduce the likelihood of unintended disconnection of RV lead 18 from IMD 14.

The electrical contacts 160, 162, 164 and 166 of the connector bore 150 are each electrically coupled to electrode terminals and circuitry within housing 15 via electrical feedthroughs 170, 172, 174 and 176 to provide electrical connection of the RV lead electrodes 26, 24, 30 and 28, respectively, to the internal IMD circuitry across the hermetically sealed IMD housing 15.

The proximal lead connector assembly 42 may correspond to an industry standard DF-4 connector, for example, having three ring connectors 130, 132, and 134 and a pin connector 135 sized and positioned to mate with respective connector block contacts 160, 162, 164 and 166. When connector block bore 150 is configured to mate with an industry standard medical electrical lead, the medical leads coupled to IMD 14 may be interchangeable. In other examples, proximal lead connector assembly 42 may be a custom assembly but different lead types may be manufactured with the same custom lead connector assembly for mating with connector block bore 150 such that different lead types, e.g., both true bipolar and integrated bipolar leads, may be coupled to IMD 14.

FIG. 3 is a conceptual diagram of an IMD system 100 including IMD 14 according to another example. In this example, IMD 14 is coupled to RA lead 16 and an integrated bipolar RV lead 118. IMD 14 may additionally be coupled to LV lead 17 as described above. In this example, integrated bipolar lead 118 includes a lead body 143 having a proximal connector assembly 142 and distal tip electrode 28, RV coil electrode 24 and SVC coil electrode 26. Tip electrode 28, RV coil electrode 24, and SVC coil electrode 26, if present, may correspond to respective tip electrode 28, RV coil electrode 24 and SVC coil electrode 26 as described above. The ring electrode 30 shown on the true bipolar RV lead 18 of FIG. 1 can be omitted from the integrated bipolar lead 118. In this case, one less conductor is required within lead body 143 compared to lead body 43 of lead 18 because ring electrode 30 is excluded. Proximal lead connector assembly 142 may be configured as an industry standard DF-4 connector or a custom connector assembly configured to mate with the same RV lead connector bore 150 (shown in FIG. 2) of connector block 12 that is configured to receive the lead connector assembly 42 of the true bipolar RV lead 18.

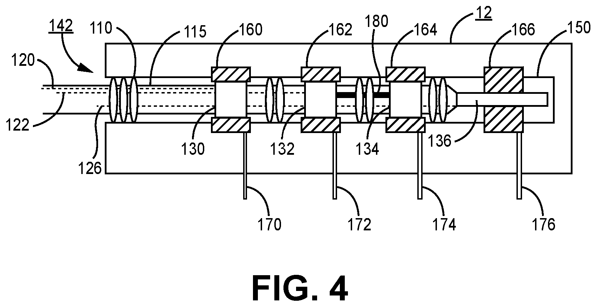

FIG. 4 is a conceptual diagram of proximal lead connector assembly 142 inserted into RV lead connector bore 150 of connector block 12 according to one example. In the case of the integrated bipolar lead 118, ring electrode 30 is excluded such that the lead conductor 124 shown in FIG. 2 extending from ring electrode 30 of RV lead 18 can be excluded from integrated bipolar RV lead 118. The size and shape of assembly body 115 may generally correspond to assembly body 115 of proximal lead connector assembly 42 of true bipolar lead 18 and conform to an industry standard or custom specification for fitting connector bore 150. The ring connector 134 corresponding to RV ring electrode 30 of true bipolar lead 18, as described above in conjunction with FIG. 2, may still be present on lead connector assembly 142 in order to conform to a lead connector industry standard or custom specification for enabling inter-changeable leads and IMDs. Ring connector 134 of lead connector assembly 142 (and lead connector assembly 42) may be referred to herein as "RV ring electrode connector" 134 for the sake of convenience even though it is recognized that the RV ring electrode 30 itself is absent from the integrated bipolar lead 118 having lead connector assembly 142. The corresponding electrical contact 164 of bore 150 may be referred to herein as a "ring electrode contact" or "RV ring electrode contact" for the sake of convenience even though the electrode contact 164 is not electrically coupled to an RV ring electrode when the integrated bipolar lead 118 is connected to IMD 14.

The ring electrode contact 164 that becomes aligned with and electrically coupled to ring electrode connector 134 is electrically coupled to an insulated electrical feedthrough 174. Electrical feedthrough 174 can thereby provide an electrically conductive pathway between ring electrode connector 134 and an electrode terminal within housing 15 that enables electrical connection of RV ring electrode 30 (when present) to sensing circuitry, therapy delivery circuitry, and impedance measurement circuitry within housing 15. In the integrated bipolar lead 118, the RV coil electrode connector 132 of lead connector assembly 142 and the RV ring electrode connector 134 may be electrically tied together, as shown conceptually in FIG. 4 by conductor 180. RV coil electrode connector 132 is electrically connected to the RV ring electrode connector 134 because RV coil electrode 24 may serve as a return electrode for bipolar sensing and pacing in the RV with RV tip electrode 28 when the integrated bipolar lead 118 is connected to IMD 14.

In some examples, lead conductor 122 extending to RV coil electrode 24 may extend to both RV coil electrode connector 132 and RV ring electrode connector 134. Electrical feedthrough 174 that couples RV ring electrode connector 134 via connector bore contact 164 to internal IMD circuitry also electrically couples internal IMD circuitry to RV coil electrode 24 in the integrated bipolar lead 118. In this way, RV coil electrode 24 may be electrically coupled to electrode terminals within housing 15 that may be switchably or selectively coupled to cardiac electrical signal sensing circuitry within housing 15 and to therapy delivery circuitry within housing 15. RV coil electrode 24 can be used for providing both bipolar sensing with tip electrode 28 and high voltage CV/DF therapy delivery. It is to be understood that switching circuitry and/or protection circuitry, such as field effect transistors (FETs), diodes, thyristors, or other semiconductor components may be included in IMD circuitry for disconnecting and/or protecting circuitry that may be coupled to an electrode terminal corresponding to RV ring electrode connector 134 for low voltage sensing, pacing and impedance measurement operations when RV coil electrode 24 is used to deliver a high voltage CV/DF shock.

Examples presented herein generally refer to determining the type of the RV lead coupled to IMD 14, e.g., either true bipolar RV lead 18 or integrated bipolar RV lead 118. It is to be understood that, additionally or alternatively, IMD 14 may be configured to receive a true bipolar or integrated bipolar lead interchangeably in one or more connector bores of connector block 12. A true bipolar or integrated bipolar lead may be advanced into different heart chambers than the RV and/or to different locations within the RV. For example, the RA lead 16 shown as a true bipolar lead (which may or may not include an SVC coil electrode) may be interchangeable with an integrated bipolar lead including the SVC coil electrode 26 carried by the RA lead body 41 and the RA tip electrode 20 without RA ring electrode 22 such that bipolar sensing and bipolar pacing in the RA may be performed between the RA tip electrode 20 and the SVC coil electrode 26.

The techniques described herein for determining a lead type, e.g., as either true bipolar or integrated bipolar, and selection of one or more operating parameters and/or one or more GUI report parameters based on the determined lead type may be applied to any lead that may be coupled to an IMD and advanced to any operative location relative to a patient's heart. The lead may be interchangeable between being a true bipolar and an integrated bipolar lead. Illustrative examples of true bipolar and integrated bipolar leads shown and described herein refer to transvenous leads advanced to position electrodes within a heart chamber, sometimes referred to as "endocardial" leads. It is contemplated, however, that techniques disclosed herein may be implemented in conjunction with transvenous or non-transvenous leads that may be connected to IMD 14 and may be configured as a true bipolar or integrated bipolar lead. Transvenous leads may be advanced to an intravascular location but remain outside the heart, e.g., within the jugular vein, internal thoracic vein, an intercostal vein, the superior epigastric vein, or the azygos, hemiazygos, or accessory hemiazygos veins, a cardiac vein or any other vein as examples. For instance LV lead 17 may be interchangeable between true and integrated bipolar lead advanced within a cardiac vein in some examples. Non-transvenous leads may include epicardial leads, intra-thoracic or substernal leads, and/or extra-thoracic or suprasternal leads that may be implanted subcutaneously or submuscularly. Examples of non-transvenous leads that may be connected to an IMD configured to operate according to aspects of the techniques disclosed herein are generally disclosed in U.S. Patent No. 10,675,478 (Marshall, et al.).

In some examples, a true bipolar lead 18 or an integrated bipolar lead 118 may be positioned for delivering ventricular pacing via the intrinsic ventricular conduction system. During normal sinus rhythm, the heartbeat is regulated by electrical signals produced by the sino-atrial (SA) node located in the right atrial wall. Each intrinsic atrial depolarization signal produced by the SA node spreads across the atria, causing the depolarization and contraction of the atria, and arrives at the atrioventricular (AV) node. The AV node responds by propagating a ventricular depolarization signal through the Bundle of His (or "His bundle") of the ventricular septum and thereafter to the Purkinje branches and the Purkinje muscle fibers of the right and left ventricles. This native conduction system including the His bundle, right and left branches (sometimes referred to as the right and left bundle branches) and the Purkinje fibers may be referred to as the "His-Purkinje conduction system" or "His-Purkinje system" and generally as the ventricular conduction system. In some examples, lead 18 or lead 118 may be positioned in the RA or RV for delivering ventricular pacing pulses using tip electrode 28 to the His bundle or the right and/or left bundle branches. For example, tip electrode 28 may be advanced into the ventricular septum from the RV to provide bundle branch pacing. Tip electrode 28 may be advanced from within the RA into the inferior end of the interatrial septum, beneath the AV node and near the tricuspid valve annulus to position tip electrode 32 in or proximate to the His bundle for delivering ventricular conduction system pacing from a RA approach.

Examples of possible positions of a lead implanted in the heart of a patient for delivering ventricular pacing via the His-Purkinje conduction system are generally disclosed in U.S. Patent No. 11,207,529 (Zhou) and in U.S. Publication No. 2022/0023640 (Zhou, et al.). It should be understood that the techniques disclosed herein are not limited to a particular heart chamber or implant location of the true bipolar or integrated bipolar lead. Aspects of the disclosed techniques may be implemented in conjunction with a lead connected to an IMD and advanced to any of a number of implant locations.

Characteristics of the bipolar cardiac electrical signal recorded from an integrated bipolar lead, e.g., lead 118, can be similar to the bipolar cardiac signal recorded from a true bipolar lead, e.g., lead 18. For example, the R-wave attendant to ventricular depolarization may have a slew rate and peak-to-peak amplitude that is similar in the cardiac electrical signal sensed by an integrated bipolar lead and by a true bipolar lead. A unipolar sensing electrode vector or configuration generally includes one electrode in the heart chamber of interest with a return electrode located remotely from the heart chamber of interest, e.g., in or on a different heart chamber or away from the heart altogether, e.g., when housing 15 or a portion thereof can be used as a return electrode for unipolar sensing. The R-wave in the unipolar sensed signal may have a lower slew rate and is generally a wider signal than the R-wave sensed from a true bipolar lead or an integrated bipolar lead because the greater inter-electrode distance between the unipolar sensing electrodes produces a more "global signal" representative of the temporal summation of the myocardial depolarization propagating through a relatively large ventricular mass. A bipolar pair of sensing electrodes, e.g., RV tip electrode 28 and RV ring electrode 30 or RV tip electrode 28 and RV coil electrode 24, having a relatively small inter-electrode distance compared to unipolar sensing electrode pairs, senses an R-wave signal representative of the depolarization of a relatively smaller more local ventricular mass.

An advantage of integrated bipolar sensing can be that one less conductor is needed in the lead body for the integrated bipolar configuration because the ring electrode may be eliminated, potentially allowing for smaller diameter lead bodies and fewer parts and assembly steps. One disadvantage of integrated bipolar sensing, however, can be slower post-shock sensing recovery time caused by electrode polarization. While characteristics of the R-wave signal sensed by a true bipolar sensing electrode vector and by an integrated bipolar sensing electrode vector can be similar, the sensed cardiac electrical signal received via an integrated bipolar sensing electrode vector and the sensed cardiac electrical signal received via a true bipolar sensing electrode vector can be different in other ways due to the relatively large surface area of the coil electrode in the integrated bipolar sensing electrode vector, relative difference in locations of the electrodes in the heart chamber, and other factors.

For example, due to the larger surface area of the RV coil electrode 24 compared to RV ring electrode 30 and, in some cases, the possibility of a greater inter-electrode spacing between RV coil electrode 24 and RV tip electrode 28 compared to the inter-electrode spacing between RV ring electrode 30 and RV tip electrode 28, the cardiac electrical signal sensed by the integrated bipolar sensing electrode vector may be more susceptible to non-cardiac noise, e.g., skeletal muscle myopotentials, 50 Hz or 60 Hz line noise, or other electromagnetic interference (EMI). When implanted, the RV coil electrode 24 is located in closer proximity to the RA than the RV ring electrode 30 so that the integrated bipolar signal may include higher amplitude far field P-waves attendant to atrial depolarizations in the signal sensed from the RV by the integrated bipolar lead 118 compared to the true bipolar lead 18. Accordingly, cross-chamber oversensing of far field signals (e.g., P-waves as false R-waves) may be more likely to occur when the sensing electrode vector is an integrated bipolar sensing electrode vector compared to a true bipolar sensing electrode vector. The bipolar signal sensed by an integrated bipolar lead may include larger T-waves (attendant to ventricular myocardial repolarization) due to the larger surface area of the elongated coil electrode 24. Accordingly, T-wave oversensing (as false R-waves) may be more likely to occur when the sensing electrode vector is an integrated bipolar sensing electrode vector compared to a true bipolar sensing electrode vector. These and other considerations may impact the optimal operating parameters used for sensing cardiac electrical signals, determining a heart rhythm and determining need for electrical stimulation therapy when an integrated bipolar lead is connected to IMD 14 compared to when a true bipolar lead is connected to IMD 14.

FIG. 5 is a conceptual diagram of IMD 14 according to some examples. The electronic circuitry enclosed within housing 15 (shown conceptually in FIG. 5 as an electrode, sometimes referred to as a "can electrode,") includes software, firmware and hardware that cooperatively monitor cardiac electrical signals, determine when an electrical stimulation therapy is necessary, and deliver therapy as needed according to programmed therapy delivery algorithms and control parameters. IMD 14 may be connected to one or more leads, such as the RA lead 16, RV lead 18 or 118, and LV lead 17, carrying electrodes 20, 22, 24, 26, 28, 30, 32 and 34 as shown in the examples of FIGs. 1 and 3, for delivering electrical stimulation pulses to the patient's heart and for sensing cardiac electrical signals.

IMD 14 includes a control circuit 80, memory 82, therapy delivery circuit 84, cardiac electrical signal sensing circuit 86 (also referred to herein as "sensing circuit" 86), telemetry circuit 88 and impedance measurement circuit 90. A power source 98 provides power to the circuitry of IMD 14, including each of the components 80, 82, 84, 86, 88 and 90 as needed. Power source 98 may include one or more energy storage devices, such as one or more rechargeable or non-rechargeable batteries. The connections between power source 98 and each of the other components 80, 82, 84, 86, 88 and 90 are to be understood from the general block diagram of FIG. 5 but are not shown for the sake of clarity. For example, power source 98 may be coupled to one or more charging circuits included in therapy delivery circuit 84 for charging holding capacitors included in therapy delivery circuit 84 and operating output circuitry for discharging the holding capacitor(s) at appropriate times under the control of control circuit 80 for producing electrical pulses according to a therapy protocol. Power source 98 is also coupled as needed to components of cardiac electrical signal sensing circuit 86 (such as sense amplifiers, analog-to-digital converters, switching circuitry, etc.), memory 82, telemetry circuit 88, and impedance measurement circuit 90 for providing an excitation signal and for recording a resulting voltage or current signal for determining an impedance.

The operating circuits shown in FIG. 5 represent functionality included in IMD 14 and may include any discrete and/or integrated electronic circuit components that implement analog and/or digital circuits capable of producing the functions attributed to IMD 14 herein. Functionality associated with one or more circuits may be performed by separate hardware, firmware and/or software components, or integrated within common hardware, firmware and/or software components. For example, signal processing circuitry configured to receive a cardiac electrical signal from a sensing electrode vector coupled to IMD 14 for processing and analysis for determining a heart rhythm and/or determining a need for an electrical stimulation therapy may include circuitry of sensing circuit 86 and control circuit 80. Signal processing and analysis of a cardiac electrical signal may therefore be performed cooperatively by sensing circuit 86 and control circuit 80 and may include operations implemented in a processor or other signal processing circuitry included in sensing circuit 85 and/or control circuit 80, which may include executing instructions stored in memory 82. Control circuit 80 may pass sensing control signals such as blanking and timing intervals and sensing threshold amplitude signals to sensing circuit 86 for sensing cardiac event signals from a cardiac electrical signal. Control circuit 80 may further process and analyze the sensed cardiac event signals and/or the cardiac electrical signal for determining when an electrical stimulation therapy is needed.

When needed, therapy delivery may be performed cooperatively by therapy delivery circuit 84 under the control of signals received from control circuit 80 for controlling the timing, pulse amplitude, pulse width, polarity, rate, electrode vector and other therapy delivery parameters used by therapy delivery circuit 84 to generate and deliver electrical stimulation pulses, which may include CV/DF pulses, cardiac pacing pulses, tachyarrhythmia induction pulses, impedance measurement pulses or any other electrical pulses delivered via electrodes 20, 22, 24, 26, 28, 30, 32 and 34 and/or housing 15.

The various circuits of IMD 14 may include an application specific integrated circuit (ASIC), an electronic circuit, a processor (shared, dedicated, or group) and memory that execute one or more software or firmware programs, a combinational logic circuit, state machine, hardware subroutine, or other suitable components or combinations of components that provide the described functionality. The particular form of software, hardware and/or firmware employed to implement the functionality disclosed herein will be determined primarily by the particular system architecture employed in the IMD and by the particular sensing, detection and therapy delivery methodologies employed by the IMD. Providing software, hardware, and/or firmware to accomplish the described functionality in the context of any modern medical device system, given the disclosure herein, is within the abilities of one of skill in the art.

Memory 82 may include any volatile, non-volatile, magnetic, or electrical non-transitory computer readable storage media, such as random-access memory (RAM), read-only memory (ROM), non-volatile RAM (NVRAM), electrically-erasable programmable ROM (EEPROM), flash memory, or any other memory device. Furthermore, memory 82 may include non-transitory computer readable media storing instructions that, when executed by one or more processing circuits, cause control circuit 80 and/or other IMD components to perform various functions attributed to IMD 14 or those IMD components. The non-transitory computer-readable media storing the instructions may include any of the media listed above.

Control circuit 80 communicates, e.g., via a data bus, with therapy delivery circuit 84 and sensing circuit 86 for sensing cardiac electrical signals, detecting cardiac rhythms, and controlling delivery of cardiac electrical stimulation therapies in response to sensed cardiac signals (or the absence thereof). Therapy delivery circuit 84 and sensing circuit 86 may be selectively electrically coupled to electrodes 20, 22, 24, 26, 28, 30, 32 and 34 carried by leads 16, 17 and 18 (or 118) and the housing 15, which may function as a common or ground electrode for sensing or cardiac pacing or as an active can electrode for delivering CV/DF shock pulses.

Sensing circuit 86 may be selectively coupled to electrodes 20, 22, 28, 30, 32 and 34 in order to monitor electrical activity of the patient's heart. Sensing circuit 86 may be coupled to RV coil electrode 24 for use in a bipolar sensing electrode vector in combination with RV tip electrode 28, in some examples. Sensing circuit 86 may monitor one or more cardiac electrical signals for sensing cardiac event signals, e.g., R-waves attendant to intrinsic ventricular myocardial depolarizations. In some examples, sensing circuit 86 may be configured to monitor two or more cardiac electrical signals for sensing cardiac event signals and may therefore include multiple sensing channels. For example, an atrial sensing channel (A sensing) 87 may receive a raw cardiac electrical signal from RA tip and ring electrodes 20 and 22. The ventricular sensing channel (V sensing) 89 may receive a raw cardiac electrical signal from the RV tip electrode 28 and RV ring electrode 30 or from the RV tip electrode 28 and RV coil electrode 24. In some examples, a third sensing channel (not shown in FIG. 5) may be provided for sensing left ventricular signals via LV lead ring electrodes 32 and 34.

Each sensing channel 87 and 89 may be configured to amplify, filter and digitize the cardiac electrical signal received from a respective sensing electrode vector to improve the signal quality for sensing cardiac event signals, such as P-waves attendant to atrial depolarizations being sensed from the RA and R-waves attendant to ventricular depolarizations being sensed from the RV and/or LV. The cardiac event detection circuitry within sensing circuit 86 can include one or more sense amplifiers, filters, rectifiers, threshold detectors, comparators, analog-to-digital converters (ADCs), timers or other analog and/or digital components.

For example, the signals received by A sensing channel 87 and V sensing channel 89 from a bipolar sensing electrode pair can be provided as differential input signals to a respective or shared pre-filter and pre-amplifier circuit for filtering of non-physiological high frequency and DC signals using a low pass or bandpass filter and amplifying the prefiltered signal by a gain of between 10 and 100. High voltage signals may be removed by protection diodes included in the pre-filter and pre-amplifier circuit. This input circuitry can provide anti-alias filtering and noise reduction prior to digitization. Each channel 87 and 89 may have a different gain and filter bandwidth of the input circuitry.

The single-ended output signal of the pre-filter and pre-amplifier circuit may be passed to an analog-to-digital converter (ADC) for sampling, e.g., at 128 or 256 Hz. The digital output of the ADC can be passed to a bandpass filter of each sensing channel having a bandpass of approximately 10 Hz to 50 Hz, or approximately 12 Hz to 40 Hz as examples, for passing cardiac electrical signals such as P-waves and R-waves typically occurring in the selected bandpass frequency range. In some examples, a wider bandpass filter of approximately 2.5 to 100 Hz may be included for passing an EGM signal to control circuit 80 for enabling waveform morphology analysis and for transmitting an EGM signal to external device 50.

In some examples, one or both of sensing channels 87 and 89 includes a notch filter to filter 50 Hz and 60 Hz noise signals. Each notch filter may be individually turned on or off in some examples. The notch filter may be implemented as hardware, firmware or software in the signal processing circuitry of sensing circuit 86 or control circuit 80. The digitized, narrow bandpass and notch-filtered signal (if notch filter is turned on) in each sensing channel 87 and 89 can be passed to a rectifier and a cardiac event detector for sensing cardiac event signals in response to the signal crossing a cardiac event sensing threshold, e.g., a P-wave sensing threshold in A sensing channel 87 or an R-wave sensing threshold in V sensing channel 89.

A cardiac event sensing threshold may be automatically adjusted by each respective sensing channel 87 and 89 under the control of control circuit 80, based on sensing threshold control parameters, such as various timing intervals including blanking and refractory periods and sensing threshold amplitude values that may be determined by control circuit 80, stored in memory 82, and/or controlled by hardware, firmware and/or software of control circuit 80 and/or sensing circuit 86. In response to sensing a cardiac event signal, e.g., a P-wave by A sensing channel 87 or an R-wave by V sensing channel 87, sensing circuit 86 may generate a sensed event signal, e.g., an atrial sensed event signal or a ventricular sensed event signal respectively, that is passed to control circuit 80.

Cardiac sensed event signals received from sensing circuit 86 by control circuit 80 can be used by control circuit 80 for determining sensed event intervals, which can be referred to as PP intervals (PPIs) or as RR intervals (RRIs). For example, an RRI can be the time interval between two ventricular sensed event signals received consecutively from sensing circuit 86 by control circuit 80. A PPI can be the time interval between two atrial sensed event signals received consecutively from sensing circuit 86 by control circuit 80. Control circuit 80 may include a timing circuit for determining PPIs and RRIs, which can include intervals between a pacing pulse and a subsequently sensed cardiac event signal. Based on PPIs and/or RRIs, control circuit 80 may detect various arrhythmias, e.g., bradycardia, asystole, atrial tachyarrhythmia, atrial fibrillation, ventricular tachyarrhythmia or ventricular fibrillation, and determine a need for an electrical stimulation therapy.

In some examples, one or more cardiac electrical signals received by sensing circuit 86 from a respective sensing electrode vector may be passed to control circuit 80 as a multi-bit digital electrogram (EGM) signal, typically a wide band filtered signal, used by control circuit 80 for morphology analysis of the cardiac signal. Time segments of EGM signals may be recorded in memory 82, e.g., in response to detecting a tachyarrhythmia, for subsequent transmission to external device 50 by telemetry circuit 88. At other times, one or more EGM signals received from sensing circuit 86 may be transmitted by telemetry circuit 88 in real time for display by external device 50 on display unit 54.

A timing circuit included in control circuit 80 may be configured to control various timers and/or counters used in setting various intervals and windows used in sensing cardiac event signals, determining time intervals between received cardiac sensed event signals, performing morphology analysis and controlling the timing of cardiac pacing pulses and/or cardioversion shock pulses generated by therapy delivery circuit 84. For instance, the timing circuit may start a timer in response to receiving ventricular sensed event signals from V sensing channel 89 for setting a ventricular pacing escape interval and for timing RRIs. Processing circuitry of control circuit 80 may compare RRIs to tachyarrhythmia detection interval thresholds for counting tachyarrhythmia intervals toward a required number of tachyarrhythmia intervals for detecting tachyarrhythmia.

Control circuit 80 may be configured to detect tachyarrhythmias according to a variety of tachyarrhythmia detection methods which may include an analysis of cardiac sensed event intervals, cardiac signal morphology, or a combination of both cardiac event interval and morphology analysis for detecting tachyarrhythmias. When an escape interval timer expires without a received cardiac sensed event signal, therapy delivery circuit 84 may deliver a pacing pulse for maintaining a desired minimum heart rate. In this way, sensing circuit 86 and control circuit 80 may operate cooperatively as signal processing circuitry configured to receive a cardiac electrical signal via a medical lead connected to IMD 14 and process the cardiac electrical signal for determining a need for an electrical stimulation therapy, which may include sensing cardiac event signals, determining cardiac sensed event intervals, and performing cardiac electrical signal morphology analysis according to operating parameters set by control circuit 80 and/or programmed by a user. As described below, based on determining if the medical lead connected to IMD 14 for sensing a cardiac electrical signal received by sensing circuit 86 is a true bipolar lead or an integrated bipolar lead, control circuit 80 may set at least one operating parameter used by the cardiac electrical signal processing circuitry for processing and analyzing the cardiac electrical signal for determining a need for an electrical stimulation therapy.

When the cardiac electrical signal processing circuitry of sensing circuit 86 and control circuit 80 determines that a therapy is needed, therapy delivery circuit 84 is configured to deliver the electrical stimulation therapy. Therapy delivery circuit 84 may include at least one charging circuit and one or more charge storage devices such as one or more capacitors for generating electrical stimulation pulses for delivery via electrodes coupled to IMD 14. Therapy delivery circuit 84 may include a high voltage therapy circuit configured to deliver high voltage CV/DF shocks and a low voltage therapy circuit configured to deliver relatively lower voltage cardiac pacing pulses. The high voltage therapy circuit can be controlled by control circuit 80 to deliver one or more high voltage shock pulses for treating a shockable rhythm detected by control circuit 80, such as ventricular tachycardia or ventricular fibrillation.

The high voltage (HV) therapy circuit may include an HV charging circuit, HV holding capacitor(s), and an HV output circuit that are operatively controlled by signals from control circuit 80 for charging and subsequently discharging the HV capacitor(s) for CV/DF shock delivery. The HV charging circuit may include one or more transformers, switches, diodes, and/or other devices for operating to charge the HV holding capacitor(s) to a desired voltage greater than the battery voltage of power source 98. Control circuit 80 may pass a charge signal to the HV therapy circuit to initiate charging and receive feedback signals from the HV charging circuit to determine when the HV holding capacitor(s) are charged to a shock voltage amplitude, e.g., corresponding to a programmed CV/DF shock energy, which may be selected based on defibrillation threshold testing or set to a nominal defibrillation energy, e.g., 10 to 20 Joules. A charge completion signal may be passed from control circuit 80 to the HV therapy circuit to terminate charging of HV holding capacitor(s) in response to determining that a desired voltage has been reached on the holding capacitor(s).

The HV holding capacitor may include one or more capacitors chargeable to a shock voltage amplitude. For example, two or three HV capacitors may be provided in series having an effective capacitance of 100 to 200 or about 150 microfarads as examples and be chargeable to deliver shocks of at least 5 Joules, at least 10 Joules or at least 20 Joules, as examples. A CV/DF shock can be delivered to the heart by discharging the HV holding capacitor(s) under the control of control circuit 80 according to signals passed to the HV output circuit, e.g., via a control bus. The HV output circuit includes switching circuitry, which may be in the form of an H-bridge. Switches in the HV output circuit are selectively biased into a conducting state from a non-conducting state by signals from control circuit 80 for discharging the HV holding capacitor(s) via a selected shock vector, e.g., via any combination of RV coil electrode 24, SVC coil electrode 26 (if present) and housing 15 serving as an active can electrode. The switching devices included in the HV output circuit may include any of a variety of semi-conductor devices such as one or more of an anode gated thyristor (AGT), metal oxide semiconductor field effect transistor (MOSFET), insulated gate bipolar transistor (IGBT), MOS-controlled thyristor (MCT), silicon-controlled rectifier (SCR) or other switching device or combination of switching devices.

Therapy delivery circuit 84 may include a low voltage therapy delivery circuit, which may include a low voltage charging circuit, one or more low voltage holding capacitors and a low voltage output circuit for generating and delivering relatively low voltage cardiac pacing pulses, e.g., cardiac pacing pulses having a pacing pulse amplitude that is 10 volts or less or 8 volts or less. Cardiac pacing pulses may be delivered by the low voltage therapy circuit in response to a pacing escape interval or other pacing timing interval expiring, as determined by control circuit 80, or in response to detection of a triggering event detected by control circuit 80. Cardiac pacing pulses may be delivered for providing bradycardia pacing, asystole pacing, ATP, CRT, post-shock pacing, etc.

The low voltage therapy circuit of therapy delivery circuit 84 may include a low voltage charging circuit including one or more low voltage holding capacitors and a low voltage output circuit that may include one or more switching devices and an output or "tip" capacitor through which the low voltage holding capacitor(s) may be discharged for delivering a pacing pulse. The low voltage charging circuit may include a charge pump for charging a low voltage holding capacitor to a pacing voltage amplitude up to a multiple of the battery voltage of power source 98, e.g., up to three or four times the battery voltage. A state machine of control circuit 80 may control charging of a low voltage holding capacitor to a programmed pacing voltage amplitude using a multiple of the battery voltage of power source 98. The low voltage holding capacitor may have a capacitance of 50 microfarads or less or as low as 10 microfarads or less.

The charged low voltage holding capacitor may be discharged via a tip capacitor by switching on an electrode selection switch after charge completion to deliver a pacing pulse to a selected cathode electrode with a return path via a selected anode electrode. The cardiac pacing pulses can be delivered as bipolar pacing pulses via a "tip-to-ring" pacing electrode vector, e.g., in the RV via RV tip electrode 28 to RV ring electrode 30 and/or in the RA via RA tip electrode 20 to RA ring electrode 22, for successfully capturing and pacing the heart. When an integrated bipolar lead, e.g., lead 118, is coupled to IMD 14, bipolar pacing pulses may be delivered via a "tip-to-coil" pacing electrode vector, e.g., from RV tip electrode 28 to RV coil electrode 24.

In some examples, in addition to being configured to deliver therapeutic electrical stimulation pulses to the patient's heart under the control circuit 80, therapy delivery circuit 84 may be controlled to deliver electrical stimulation pulses for inducing tachyarrhythmia, e.g., T-wave shocks or trains of induction pulses, upon receipt of a programming command from external device 50 (FIG. 1A) by telemetry circuit 88, e.g., during IMD implant or follow-up testing procedures. In some examples, therapy delivery circuit 84 may be configured to deliver excitation signals for measuring lead impedance in cooperation with impedance measurement circuit 90.

Impedance measurement circuit 90 is configured to measure the impedance between pairs of electrode terminals 92 that can be coupled to the respective electrodes, e.g., electrodes 20, 22, 24, 26, 28, 30, 32, 34 carried by a lead coupled to IMD 14 via connector block 12 (and in some cases the "can electrode" shown as housing 15). Electrode terminals 92 may be electrically coupled to electrical contacts within connector block 12 (shown in FIGs. 1-4). As described above in conjunction with FIGs. 2 and 4, electrical contacts in each connector bore of connector block 12 may be electrically coupled to the circuitry of IMD 14 via electrical traces, feedthroughs and/or other conductive elements for establishing electrical connection with respective electrical connectors of each lead connector assembly of the leads 16, 17 and 18 (or 118) received by a respective bore of connector block 12. An impedance signal measured between a selected pair of electrode terminals 92 may be passed from impedance measurement circuit 90 to control circuit 80 for use in determining a lead type connected to IMD 14 as disclosed herein and for monitoring for lead related issues such as lead fractures, insulation breach, poor lead connection within connector block 12, or other functional lead issues. As described below, based on a determined lead type as being either true bipolar or integrated bipolar, control circuit 80 may select and apply one or more operating parameters for controlling IMD 14 operations and/or for use in generating reports by external device 50 for display in a GUI.

Impedance measurement circuit 90 may include a multiplexer or other switching circuitry for coupling selected terminals 92, each corresponding to an electrode that may be available on a given lead 16, 17 or 18 (or 118) when connected to IMD 14. Impedance measurement circuit 90 may include a drive circuit for generating an excitation current or voltage signal that can be injected across an excitation pair of electrode terminals 92 for measuring the resulting impedance between a recording pair of electrode terminals 92. In some examples, the excitation pair of electrode terminals and the recording pair of electrode terminals may be the same and in other examples two different pairs of electrode terminals 92 are used for injecting the excitation signal and for recording the resulting voltage and/or current signal.

The excitation current signal or excitation voltage signal may be applied as a subthreshold signal having a pulse amplitude and/or pulse width that is less than the pacing capture threshold of the patient's heart. In this way, an impedance measurement may be obtained without causing an evoked response (depolarization) of cardiac tissue. The excitation signal may include monophasic or biphasic pulses having a pulse amplitude of less than 0.5 volts or less than 0.25 volts with a pulse width of 10 to 50 microseconds or about 25 to 30 microseconds, as examples. However, other subthreshold signals may be applied as excitation signals. A "subthreshold" signal refers to a signal having a pulse energy below the capture threshold of the heart so that an evoked depolarization of the myocardial tissue does not occur when the excitation signal is injected by impedance measurement circuit 90.

Impedance measurement circuit 90 may include a sampling circuit for sampling the resulting voltage and/or current signal from a recording pair of terminals selected from terminals 92. The sampled signals may be used by processing circuitry of impedance measurement circuit 90 to derive an impedance signal (which may take into account the current or voltage of the delivered excitation signal). Impedance measurement circuit 90 may receive a resulting voltage or current signal from a recording pair of electrode terminals 92 in response to applying the excitation signal and may use the resulting signal as an impedance measurement signal or convert the resulting signal to an impedance signal by determining the impedance in ohms based on the applied excitation signal and recorded signal. Examples of circuitry and techniques for measuring the electrical impedance of a lead and electrode pair coupled to IMD 14 are generally disclosed in U.S. Patent No. 5,534,018 (Wahlstrand, et al.) and U.S. Patent No. 8,996,111 (Marshall, et al.).

The techniques disclosed herein may be implemented in conjunction with a variety of circuits and techniques for performing impedance measurements. In some examples, the impedance measurement circuit 90 may share components with control circuit 80, therapy delivery circuit 84 and/or electrical sensing circuit 86. While impedance measurement circuit 90 is shown conceptually as a separate functional block in the diagram of FIG. 5, impedance measurement circuit 90 is not necessarily a dedicated circuit and may include a combination of components and functionality that is shared between therapy delivery circuit 84, sensing circuit 86 and/or control circuit 80 for cooperatively performing an impedance measurement.

For example, an excitation pulse may be generated by therapy delivery circuit 84 under the control of processing and control circuitry included in impedance measurement circuit 90 and/or control circuit 80. The excitation pulse may be controlled to have a starting voltage amplitude and processing circuitry of impedance measurement circuit 90 (or control circuit 80) may determine the voltage change on a holding capacitor of therapy delivery circuit 84 at the end of the excitation pulse. The voltage of the holding capacitor may be sampled at the beginning and end of the excitation pulse width. The discharge of the holding capacitor during the excitation pulse, from a starting voltage to an ending voltage, is inversely correlated to the electrode and lead impedance coupled to the respective terminals 92. The greater the voltage change, the lower the impedance.

In other examples, impedance measurement circuit 90 may control therapy delivery circuit 84 to generate a voltage pulse between first and second electrode terminals of terminals 92. Impedance measurement circuit 90 may measure a resulting current and derive an impedance value therefrom, e.g., based upon the voltage amplitude of the excitation pulse and the measured amplitude of the resulting current. In still other examples, the impedance measurement circuit 90 may control therapy delivery circuit 84 to deliver a current pulse between first and second electrode terminals of terminals 92. The impedance measurement circuit 90 may measure a resulting voltage across the first and second electrode terminals, and control circuit 80 may derive an impedance value therefrom, e.g., based upon the current amplitude of the pulse and the measured amplitude of the resulting voltage. The impedance measurement circuit 90 may include circuitry for measuring amplitudes of resulting currents or voltages, such as sample and hold circuitry. The impedance measurement circuit 90 may be configured to measure impedance with a relatively high resolution of 20 ohms, 10 ohms, 5 ohms or 1 ohm in various examples. The impedance may be measured over a range of 0 to 2,000 ohms or 0 to 1,000 ohms or 0 to 1500 ohms using a resolution of 1 to 30 bits per ohm or about 20 bits per ohm, as examples. As described below, an impedance measurement between an RV ring electrode terminal and an RV coil electrode terminal of terminals 92 may be determined for detecting the type of lead connected to IMD 14. This "ring-to-coil" impedance measurement may be measured in a range of 0 to 300 ohms or 0 to 200 ohms, as examples, with a resolution of 1 to 5 bits per ohm as examples.

Telemetry circuit 88 includes a transceiver and antenna for communicating with external device 50 (shown in FIGs. 1 and 2) using RF communication or other communication protocols as described above. Operating parameters utilized by control circuit 80 for sensing cardiac event signals, detecting arrhythmias, and controlling therapy delivery may be programmed into memory 82 via telemetry circuit 88. Under the control of control circuit 80, telemetry circuit 88 may receive downlink telemetry from and send uplink telemetry to external device 40.

Control circuit 80 may pass digital data signals to telemetry circuit 88 for modulation and transmission to external device 50. External telemetry unit 58 may receive the modulated signals for demodulation and pass the data to processor 52. Processor 52 may use the data in generating GUIs for display by display unit 54. Control circuit 80 and telemetry circuit 88 may cooperatively transmit a detected lead type and/or associated commands to external device 50 for properly displaying sensing and pacing electrode vectors and other data relevant to the type of detected lead, e.g., either a true bipolar or an integrated bipolar lead, as further described below.

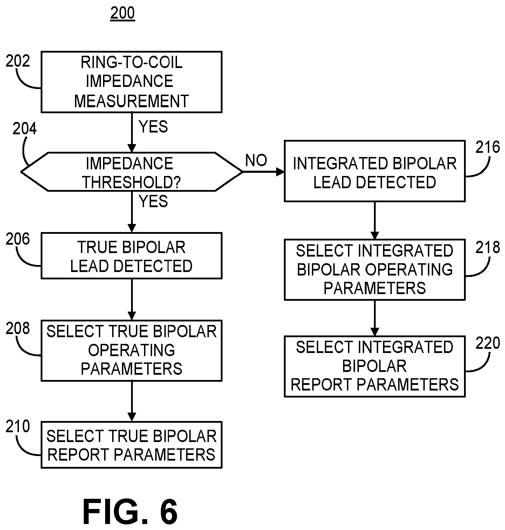

FIG. 6 is a flow chart 200 of a method that may be performed by IMD 14 for determining a connected lead type as being either a true bipolar lead or an integrated bipolar lead. The true bipolar lead can include a tip electrode, a ring electrode and a coil electrode or any two small surface area electrodes for bipolar sensing and one high surface area electrode for CV/DF therapy delivery. The integrated bipolar lead can include a tip electrode (or any small surface area electrode) and a coil electrode for sensing with the tip electrode and for CV/DF therapy delivery. A ring electrode (or second small surface area electrode) is excluded from the integrated bipolar lead. At block 202 control circuit 80 controls impedance measurement circuit 90 to perform an impedance measurement between one of electrode terminals 92 that is electrically coupled to a ring electrode contact in the connector block 12 of IMD 14 and another electrode terminal 92 that is connected to a coil electrode contact of the same connector bore of connector block 12.

For example, with reference to FIGs. 2 and 4, the impedance measurement may be performed to determine the impedance between the RV coil electrode contact 162 and the RV ring electrode contact 164 of the connector bore 150 in connector block 12. This impedance measurement can be referred to as a "ring-to-coil" impedance measurement even though a lead coupled to IMD 14 in connector bore 150 may not be carrying a ring electrode. With reference to RV lead 118, as shown in FIG. 4, RV the ring electrode connector 134 and the RV coil electrode connector 132 of proximal connector assembly 142 may be electrically tied together (e.g., by conductor 180). Both the RV coil electrode terminal (coupled to connector bore contact 162) and the RV ring electrode terminal (coupled to connector bore contact 164) of terminals 92 are electrically coupled to RV coil electrode 24 when the integrated bipolar lead 118 is connected to IMD 14.

The ring-to-coil impedance measurement may be performed at block 202 upon implantation of IMD 14 in a patient. IMD 14 may be configured to detect implantation based on impedance measurements, based on programming tachyarrhythmia detection "on," or other indicators that the IMD 14 is implanted in a patient. Additionally or alternatively, the ring-to-coil impedance measurement may be performed at block 202 at one or more predetermined time intervals after implant detection, e.g., 30 minutes, 60 minutes, and/or 120 minutes, with no limitation intended. The ring-to-coil impedance measurement at block 202 may be repeated at scheduled times of day, e.g., once nightly, and/or when any other lead impedance measurement, e.g., an RV tip to RV coil impedance measurement or an RV tip to RV ring impedance measurement, indicates a change in impedance that may be due to a lead replacement procedure.

Impedance measurement circuit 90 may perform the ring-to-coil impedance measurement over a narrower impedance range with higher resolution than other lead impedance measurements that may be performed, e.g., for monitoring for lead-related issues or other device diagnostic or impedance monitoring purposes. For example, impedance measurement circuit 90 may be configured to measure the ring-to-coil impedance over range of 0 to 300 ohms or 0 to 200 ohms or 0 to 150 ohms or 0 to 100 ohms, as examples, with a resolution of 1 to 5 bits per ohm. In one example, the ring-to-coil impedance is measured over a range of 0 to 200 ohms with a 1 bit per ohm resolution. In comparison, impedance measurement circuit 90 may perform other impedance measurements between other terminals 92 as needed over a higher impedance range, e.g., up to 1000 ohms, 2000 ohms or 3000 ohms, which may be measured with a lower resolution, e.g., 5 to 30 ohms per bit.

At block 204, control circuit 80 compares the ring-to-coil impedance measurement to a threshold impedance. The threshold impedance may be between 20 and 70 ohms and may be between 40 to 60 ohms or about 50 ohms as examples. The threshold impedance is selected to discriminate the relative difference in impedance between a ring-to-coil impedance measurement of an integrated bipolar lead and ring-to-coil impedance measurement of a true bipolar lead. The ring-to-coil impedance of a true bipolar lead, which includes separate insulated conductors extending from a ring electrode and an elongated coil electrode each carried by the true bipolar lead body, may be in the range of 50 to 200 ohms. The ring-to-coil impedance of an integrated bipolar lead, which excludes a ring electrode and may include a conductor that electrically couples the lead connector assembly coil electrode contact with the lead connector assembly ring electrode contact may be less than 50 ohms, less than 20 ohms and even less than 10 ohms in some examples. The ring-to-coil impedance measurement obtained by impedance measurement circuit 90 when an integrated bipolar lead is coupled to IMD 14 may be on the order of 5 ohms, for example.

When the ring-to-coil impedance is greater than a threshold impedance, e.g., greater than 50 ohms, control circuit 80 detects a true bipolar lead at block 206. In response to the true bipolar lead being detected, control circuit 80 selects operating parameter settings at block 208 and report parameters at block 210 that are appropriate for sensing and pacing functions using a bipolar tip-to-ring electrode vector. When the ring-to-coil impedance is less than (or equal to) the threshold impedance at block 204, control circuit 80 detects an integrated bipolar lead at block 216. In response to the integrated bipolar lead being detected, control circuit 80 selects operating parameter settings at block 218 and report parameters at block 220 that are appropriate for sensing and pacing functions using an integrated bipolar tip-to-coil electrode vector.

It is to be understood that when the ring-to-coil impedance measurement is not the first measurement since IMD implant, control circuit 80 may determine that, since the most recent preceding impedance measurement, the ring-to-coil impedance is still greater than the threshold impedance or still less than the threshold impedance, indicating that a previously determined lead type has not changed. In this case, control circuit 80 does not need to reselect operating parameters and report parameters based on the same lead type still being detected since a most recent previous ring-to-coil impedance measurement. The previously selected operating parameters and report parameters corresponding to the previously detected lead type may remain in effect when a change from a ring-to-coil impedance measurement being less than the threshold impedance to greater than the threshold impedance, or vice versa, is not detected.

At least some of the operating parameters that are selected at block 218 and/or at block 220 are selected due to the differences in the characteristics of integrated bipolar sensed cardiac electrical signals and true bipolar sensed cardiac electrical signals. In some examples, a true bipolar lead may be the expected default lead for use with IMD 14. As such, control circuit 80 may be pre-programmed or initialized to operating parameters and report parameters corresponding to a true bipolar lead as default parameter settings that may be left unchanged at block 208 or 210.

One operating parameter that is selected is the bipolar sensing/pacing vector as being either a tip-to-ring vector for the true bipolar lead detection or the tip-to-coil sensing vector for the integrated bipolar lead detection. Control circuit 80 may control switching circuitry of sensing circuit 86 and/or therapy delivery circuit 84 for coupling corresponding electrode terminals 92 to input and output circuits, respectively, as needed for selecting the tip-to-ring electrode vector or tip-to-coil electrode vector for sensing and/or pacing therapy delivery based on the detected lead type. Other operating parameter settings that are selected at block 208 or 218 that are dependent on whether a true bipolar or integrated bipolar lead is detected may include filtering and/or blanking times applied to the cardiac signal sensed by sensing circuit 86 via the tip-to-ring or tip-to-coil sensing electrode vector. Yet another operating parameter setting that may be selected at block 208 or block 218 by control circuit 80 may be the ADC range of sensing circuit 86 for the V sensing channel 89. The bipolar signal sensed from tip-to-coil using an integrated bipolar lead may have greater peak-to-peak amplitude than the signal sensed tip-to-ring using a true bipolar lead. As such, control circuit 80 may select the ADC amplitude range based on the detected lead type. The ADC amplitude range may be selected to be between + 6 millivolts and ± 14 millivolts or between + 8 millivolts and ± 12 millivolts, as examples. The ADC range may be adjusted to a higher input range in response to detecting an integrated bipolar lead to reduce the likelihood of an over-range R-wave signal waveform.

The operating parameters selected at blocks 208 and 218 may additionally or alternatively include enabling or disabling oversensing detection algorithms and/or setting different oversensing detection criteria used by signal processing circuitry of sensing circuit 86 and/or control circuit 80 for detecting cardiac event oversensing (e.g., oversensing of P-waves and/or T-waves as false R-waves by ventricular sensing channel 89) and/or for detecting noise contamination of the cardiac electrical signal or non-cardiac noise oversensing (e.g., oversensing skeletal muscle or diaphragmatic myopotential signals or EMI as false R-waves).

The operating parameters selected at block 208 or 218 may additionally or alternatively include setting an impedance threshold range for comparing bipolar impedance measurements made using a bipolar sensing electrode pair that includes a tip electrode, e.g., RV tip electrode 28. The normal bipolar impedance measurement range for a true bipolar (tip-to-ring) impedance may be set differently at block 208 than the normal impedance measurement range for an integrated bipolar (tip-to-coil) impedance. A maximum and/or minimum bipolar impedance threshold limit may be set differently depending on whether a true bipolar or integrated bipolar lead is detected. The normal bipolar impedance range may be applied by control circuit 80 to the bipolar impedance measurement obtained from the RV lead coupled to IMD 14 for use in monitoring for lead-related issues, such as a conductor fracture, insulation breach, or improper lead connection.

A normal true bipolar lead tip-to-ring impedance measurement may be in the range of 100 ohms to 300 ohms. A normal integrated bipolar lead tip-to-ring impedance measurement (that may be performed between the same tip electrode terminal and ring electrode terminal of terminals 92 as when a true bipolar lead is connected) may be relatively lower, e.g., 50 to 100 ohms, due to the large surface area of the elongated coil electrode that is electrically coupled to the ring electrode terminal. Control circuit 80 may store impedance measurements in memory 82 and may flag out of range impedance measurements in memory 82. The stored impedance measurement data, including flagged out of range measurements, may be transmitted to external device 50 to alert a clinician that a lead issue may exist, which may require reprogramming of IMD 14 to eliminate an electrode and conductor pathway associated with an out of range impedance and/or may require medical intervention, e.g., to replace the lead, or other lead-related issue mitigation.

In some instances, control circuit 80 may be configured to automatically select a different sensing electrode vector and/or therapy delivery electrode vector in response to detecting a lead issue based on an impedance measurement. When a bipolar impedance measurement causes control circuit 80 to detect a false positive lead issue, control circuit 80 may switch an electrode vector selection from the bipolar electrode vector (either tip-to-ring or tip-to-coil) to a different sensing and/or therapy delivery electrode vector available from the leads coupled to the IMD 14, which could be less optimal for sensing and/or therapy delivery than the bipolar electrode vector that may still be fully functional and available.

If a single "normal impedance range" is applied to impedance measurements obtained between the RV tip electrode terminal and the RV ring electrode terminal of terminals 92 (or between the RV tip electrode terminal and the RV coil electrode terminal of terminals 92), a lead impedance measurement may falsely appear to be out of range due to the normal difference that exists between the tip-to-ring impedance measurement when a true bipolar lead is connected to IMD 14 compared to the tip-to-ring impedance measurement when an integrated bipolar lead is connected to IMD 14. A bipolar tip-to-ring impedance measurement from RV lead 18 or RV lead 118 could be flagged as a false positive lead issue and/or left unflagged as a false negative lead issue. False positive lead issues identified based on tip-to-ring (or tip-to-coil) impedance measurements may lead to unnecessary evaluation and troubleshooting by a clinician, technician or other medical personnel. False negative lead issues that are not identified based on bipolar lead impedance measurements may result in a lead issue being unidentified and going unresolved. An unresolved lead issue could result in under- or oversensing of cardiac arrhythmias and/or ineffective or reduced effectiveness of delivered electrical stimulation therapies that use the bipolar pacing electrode vector. Accordingly, by setting a unique normal impedance measurement range in response to detecting an integrated bipolar lead, different from the normal impedance measurement range applied to true bipolar lead impedance measurements, false positive and false negative reports of lead-related issues can be reduced.

At block 210 or block 220, control circuit 80 may select report parameters that are relevant to the detected lead type for generating reports and displaying data on external device 50 (or a remote patient management system) relating to sensing and pacing electrode vector selections, lead impedance monitoring, delivered therapies, and so on. For example, data buffers allocated in memory 82 for storing tip-to-ring impedance measurements, tip-to-ring capture thresholds, and tip-to-ring pacing history may be omitted or reallocated to tip-to-coil impedance measurements, capture thresholds, pacing history or the like when an integrated bipolar lead is detected. Data acquired by control circuit 80 and stored in memory 82 may be allocated to separately store bipolar tip-to-ring related data from bipolar tip-to-coil related data for device diagnostic and reporting purposes when a true bipolar lead is detected.

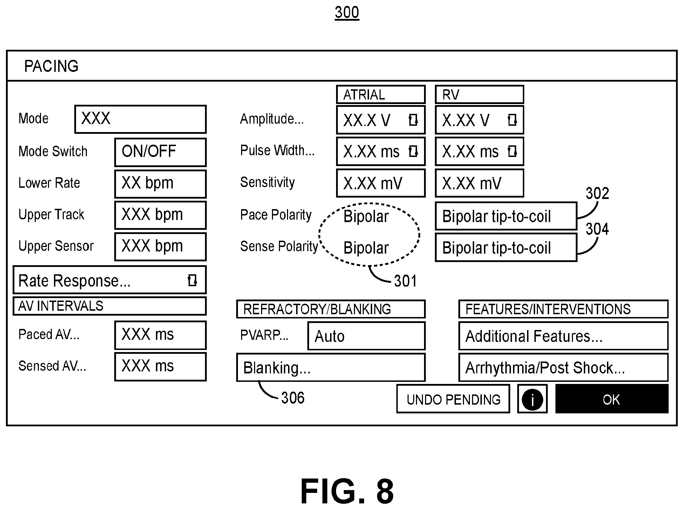

The selection of report parameters at block 210 or block 220 may include transmitting a signal indicative of the detected lead type by telemetry circuit 88. External device telemetry unit 58 may receive the transmitted signal. External device processor 52 may respond to the received lead type notification by generating GUI(s) for display by display unit 56 that include reports, displayed data and/or displayed data labels that are selected to correspond appropriately to the detected lead type. Examples of GUIs that may be generated for display by display unit 54 with at least some report parameters, data, and/or data labels selected based on the lead type detected by IMD control circuit 80 are described below in conjunction with FIGs. 8-11.

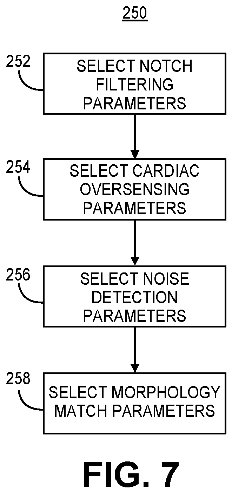

FIG. 7 is a flow chart 250 of a method that control circuit 80 may perform for selecting operating and report parameters in response to detecting an integrated bipolar lead connected to the IMD 14 (e.g., as described above with reference to FIG. 6). The operations performed by IMD 14 described in conjunction with FIG. 7 may correspond to the selection of operating parameters and report parameters performed at blocks 218 and 220 of FIG. 6. At block 252, control circuit 80 may enable a notch filter for filtering the bipolar cardiac electrical signal sensed from the tip-to-coil electrode vector that is selected as the sensing electrode vector in response to detecting the integrated bipolar lead. The cardiac electrical signal sensed from a tip-to-coil electrode vector may be more susceptible to 50 Hz and 60 Hz line noise than the bipolar signal sensed from a tip-to-ring electrode vector due to the relatively high surface area of the elongated coil electrode compared to a ring electrode. As described above in conjunction with FIG. 5, sensing circuit 86 may include a notch filter that may be implemented in hardware, firmware or software for filtering 50 Hz and 60 Hz noise from the sensed cardiac electrical signal. The notch filter may be enabled or disabled (e.g., turned "on" or "off") by control circuit 80 and may be programmably enabled or disabled by a user interacting with external device 50 in some examples. In some examples, the notch filter is enabled by control circuit 80 when an integrated bipolar lead is detected to filter 50 Hz and 60 Hz noise from the bipolar cardiac electrical signal.

The notch filter may be permanently enabled at block 252 such that it operates to provide notch filtering of the sensed tip-to-coil signal at all times. In other examples, the notch filter may be enabled on an as needed basis at block 252 by enabling noise detection for triggering notch filtering of the sensed tip-to-coil signal. As described below (in conjunction with block 256), noise detection may be enabled or disabled based on a detected lead type. When noise is detected in the cardiac electrical signal, or oversensing of noise is suspected, control circuit 80 may turn on the notch filter. Various methods may be used for detecting noise in the sensed cardiac signal or determining suspected noise oversensing from the sensed cardiac electrical signal. Example noise detection methods are generally disclosed in U.S. Patent No. 7,774,049 (Ghanem, et al.) and in U.S. Patent No. 7,783,354 (Gunderson). Any signal noise detection method or suspected noise oversensing detection method may be implemented in IMD 14 for execution by control circuit 80 for enabling notch filtering of the sensed cardiac electrical signal when an integrated bipolar lead is detected.

Control circuit 80 may select a report parameter at block 252 by generating a signal for transmission by telemetry circuit 88 that indicates that notch filtering of the bipolar sensed cardiac electrical signal is turned on. In other examples, telemetry circuit 88 may transmit a notification signal indicating the determination of an integrated bipolar lead, and external device processor 52 may be configured to recognize that notch filtering of the bipolar sensed signal is automatically enabled by IMD 14 (or enabled on a triggered basis when noise is detected or suspected) when an integrated bipolar lead is detected. Based on this recognition, external device processor 52 may generate a GUI for display by display unit 54 that may exclude a user programming option for enabling or disabling notch filtering in some examples. In other examples, the GUI may be generated to include a user programmable option for enabling or disabling notch filtering with a notification that notch filtering is currently on and/or that enabling of notch filtering is the recommended setting for the detected integrated bipolar lead.

In contrast, when a true bipolar lead is detected at block 206 of FIG. 6, control circuit 80 may omit enabling notch filtering in selecting true bipolar operating parameters at block 208 because the true bipolar sensed cardiac signal is generally less susceptible to 50 and 60 Hz noise. Notch filtering may be turned off by default when a true bipolar lead is detected by control circuit 80 but may be enabled by a user-entered programming command received from external device 50 in some examples. Some patients may live or work in an environment that may make them more prone to 50 or 60 Hz noise exposure that warrants programming notch filtering on even when a true bipolar lead is connected to IMD 14. In still other examples, notch filtering may be enabled to operate continuously when an integrated bipolar lead is detected and may be enabled to operate on a triggered basis in response to noise detection by control circuit 80 when a true bipolar lead is detected.

When a true bipolar lead is detected, a user programmable option for enabling or disabling notch filtering may be included in a GUI. In some examples, notch filtering may be turned off by default or set by control circuit 80 to be turned off in response to true bipolar lead detection at block 206. A report parameter signal may be transmitted by telemetry circuit 88 at block 210 indicating that notch filtering is turned off. In response to receiving the report parameter signal or in response to a notification of a true bipolar lead determination, external device processor 52 may control display unit 54 to display a GUI indicating that notch filtering is turned off and/or that the recommended setting for notch filtering is "off."

Returning to FIG. 7, at block 254, control circuit 80 may enable cardiac oversensing detection in response to determining that an integrated bipolar lead is connected to IMD 14. Cardiac oversensing refers to oversensing of other cardiac event signals. To illustrate, V sensing channel 89 (FIG. 5) can be configured to sense R-waves in response to R-wave sensing threshold crossings by the bipolar cardiac electrical signal. P-waves and/or T-waves, however, may have a large enough amplitude in some instances to cross the R-wave sensing threshold resulting in an oversensed cardiac event signal. The sensing circuit 86 may pass a false ventricular sensed event signal to control circuit 80. The bipolar signal sensed by an integrated bipolar lead may include larger T-waves than the bipolar signal sensed by a true bipolar lead due to the larger surface area of the elongated coil electrode. The bipolar signal sensed by an integrated bipolar lead may include larger far field P-wave signals than the bipolar signal sensed by a true bipolar lead due to the larger surface area and the closer proximity to the atria of the RV coil electrode, e.g., coil electrode 24 shown in FIG. 2. The larger T-waves and/or larger far field P-waves that may be present in the bipolar signal sensed by an integrated bipolar lead advanced into the RV can increase the likelihood of cardiac event oversensing by V sensing channel 89.

As such, at block 254 in response to detecting the integrated bipolar lead, control circuit 80 may be configured to enable a cardiac event oversensing detection algorithm and/or adjust cardiac event oversensing detection criteria or another operating parameter used by signal processing circuitry for determining when cardiac event oversensing is occurring. Control circuit 80 may enable a P-wave oversensing detection algorithm and/or a T-wave oversensing detection algorithm in response to detecting the integrated bipolar lead. The cardiac event oversensing detection algorithm(s) may include evaluating RRIs determined between consecutive ventricular sensed event signals received from sensing circuit 86 to detect alternating short and long intervals which may be indicative of P-R-P or R-T-R sequences of sensed events.

Cardiac event oversensing detection algorithm(s) may additionally include analysis of the peak amplitudes of the sensed event signals. For example, a low-high-low peak amplitude sequence may indicate a P-R-P or T-R-T event sequence. The cardiac event oversensing detection algorithm(s) may additionally or alternatively include a waveform morphology analysis. For example, control circuit 80 may be configured to compare the waveform of a sensed signal to a previously established R-wave morphology template to determine a morphology match score, e.g., using a wavelet transform or other waveform matching or correlation analysis. When the morphology match scores are alternating, e.g., a high-low-high morphology match score sequence, the sensed signals may correspond to R-P-R or R-T-R sequences. A combination of sensed event intervals, amplitudes and/or morphology analysis may be performed by signal processing circuitry included in control circuit 80 to detect cardiac event oversensing.

Any of a variety of cardiac event oversensing techniques may be implemented in IMD 14, which may be selectively enabled by control circuit 80 when an integrated bipolar lead is detected. Example techniques for T-wave oversensing detection are generally disclosed in U.S. Patent No. 8,942,795 (Gunderson, et al.). Example techniques for P-wave oversensing detection are generally disclosed in U.S. Patent No. 11,135,441 (Zhang, et al.). When a true bipolar lead is detected, control circuit 80 may turn off a cardiac event oversensing detection algorithm in some examples (e.g., at block 208 of FIG. 6). A user may program cardiac event oversensing detection on or off based on individual patient need when a true bipolar lead is detected. In some examples, control circuit 80 may enable T-wave oversensing detection and disable P-wave oversensing detection in response to detecting a true bipolar lead because the likelihood of far-field P-wave oversensing from a bipolar signal sensed by a true bipolar lead in the RV is relatively low.

In other examples, in addition to or alternatively to turning a cardiac event oversensing algorithm on or off, one or more operating parameters used to control detection of cardiac event oversensing by the algorithm may be set by control circuit 80 at block 254 when an integrated bipolar lead is detected (or block 208 of FIG. 6 when a true bipolar lead is detected). For example, one or more cardiac signal features determined by IMD signal processing circuitry for detecting cardiac event oversensing may be selected based on the type of medical lead detected and/or one or more thresholds or other criteria applied to determined cardiac signal features may be selected by control circuit 80. The cardiac event oversensing detection method that is performed may be different when an integrated bipolar lead is detected compared to when a true bipolar lead is detected.

For instance, T-wave oversensing detection may be turned on when either a true or integrated bipolar lead is detected, but the processing and analysis performed for detecting T-wave oversensing from a true bipolar signal vs. an integrated bipolar signal may be different. To illustrate, control circuit 80 may be configured to determine the difference between the maximum peak amplitudes of consecutive pairs of sensed event signals for detecting cardiac event oversensing. Control circuit 80 may apply a difference threshold to the determined peak amplitude difference for detecting suspected oversensing sequences of alternating R-T-R or T-R-T (or P-R-P or R-P-R) signals. Control circuit 80 may select a smaller difference threshold to be applied to the peak amplitude differences determined from the bipolar signal sensed by an integrated bipolar lead (tip-to-coil) and a relatively larger difference threshold to be applied to peak amplitude differences determined from a true bipolar (tip-to-ring) sensed signal. A smaller difference threshold may be used in detecting cardiac event oversensing in response to detecting an integrated bipolar lead because the P-wave and T-wave peak amplitudes may be larger in the integrated bipolar sensed signal, closer to the peak amplitudes of true R-waves. In various examples, the thresholds, ranges or other values or criteria selected by control circuit 80 at block 254 for applying to signal features determined by IMD signal processing circuitry from the integrated bipolar tip-to-coil signal may be different than the thresholds, ranges or other values or criteria selected by control circuit 80 at block 208 (FIG. 6) for applying to signal features determined from the true bipolar tip-to-ring signal for cardiac event oversensing detection. Example signal features that the selected thresholds or other criteria are applied to may include, with no limitation intended, morphology matching scores, slew rates, peak amplitudes, signal widths, signal areas, peak polarity and cardiac event intervals.

Furthermore, signal features that provide a high confidence in detecting cardiac event oversensing from an integrated bipolar signal may be different than the signal features that provide a high confidence in detecting cardiac event oversensing from a true bipolar signal. As such, control circuit 80 may enable two different cardiac event oversensing detection algorithms depending on the detected lead type that rely on the determination and analysis of different cardiac signal features by the IMD signal processing circuitry. To illustrate, control circuit 80 may select a first cardiac event oversensing detection algorithm that includes determining and analyzing maximum peak amplitudes and sensed event intervals, e.g., when a true bipolar lead is detected, and may select a second, different cardiac event oversensing detection algorithm that includes determining and analyzing sensed event intervals and morphology matching scores when an integrated bipolar lead is detected. Control circuit 80 may be configured to turn on or select a cardiac event oversensing detection algorithm based on the detected lead type that provides a desired confidence in detecting cardiac event oversensing while minimizing or reducing necessary processing burden and power requirements for oversensing detection.

It is to be understood that when control circuit 80 selects an operating parameter in response to a detected lead type by enabling a cardiac event oversensing detection algorithm, the cardiac event oversensing detection algorithm may not be performed on a beat-by-beat basis twenty-four hours a day. The cardiac event oversensing detection algorithm may be performed at scheduled times and/or in response to triggering events, e.g., when a threshold number of ventricular sensed event signals are received from sensing circuit 86 at tachyarrhythmia intervals. The cardiac event oversensing detection algorithm may be performed when turned on and a tachyarrhythmia interval count is trending up to avoid falsely detecting a tachyarrhythmia due to cardiac event oversensing, for example.

Additionally or alternatively, control circuit 80 may adjust R-wave sensing control parameters at block 254 to promote avoidance of cardiac event oversensing. For example, control circuit 80 may increase the programmed R-wave sensitivity setting, to avoid or reduce the likelihood of cardiac event oversensing. The sensitivity setting is the lowest amplitude that the R-wave sensing threshold can be set to during a cardiac cycle for sensing an R-wave in response to the cardiac electrical signal crossing the R-wave sensing threshold. By increasing the sensitivity setting, the sensing circuit 86 is less sensitive to oversensing far field P-waves or T-waves as false R-waves.

In some examples, R-wave sensing control parameters set by control circuit 80 at block 254 to promote avoidance of T-wave or far-field P-wave oversensing may include various operating parameters used by sensing circuit 86 for adjusting the R-wave sensing threshold amplitude. One or more decay times, drop time intervals, amplitudes (which may be set as a percentage of a maximum peak amplitude of the most recently sensed signal) and/or the sensitivity may be set by control circuit 80 based on the determined lead type.

Control circuit 80 may select one or more report parameters that may be transmitted by telemetry circuit 88 at block 254 when control circuit 80 selects or adjusts a setting for avoiding or detecting cardiac event oversensing based on the detected integrated bipolar lead type, including any of adjusting an R-wave sensing control parameter, enabling cardiac event oversensing detection algorithm(s), selecting signal features to be determined for cardiac event oversensing detection and/or selecting thresholds, ranges, signal feature values or other oversensing detection criteria at block 254. Similarly, when a cardiac event oversensing related control parameter is adjusted at block 208 of FIG. 6 in response to detecting a true bipolar lead, control circuit 80 may select one or more relevant report parameters at block 210 of FIG. 6. In various examples, telemetry circuit 88 may transmit a notification indicating whether far-field P-wave oversensing detection and/or T-wave oversensing detection is turned on or off, transmit selected cardiac event detection operating parameters, and/or transmit a recommended setting for turning on or off cardiac event oversensing detection and/or any recommended settings for thresholds or other criteria used for detecting cardiac event oversensing. Adjustments to any R-wave sensing threshold control parameters may also be reported. External device 50 may receive a transmitted signal relating to selected and/or recommended operating parameter settings for controlling cardiac event oversensing detection or controlling R-wave sensing for display in a GUI by display unit 54.

It is to be understood that in some examples external device processor 52 may be configured to select reports, report parameters, data labels, programmable operating parameter settings etc. for display in a GUI in response to a notification of a detected lead type received from IMD 14 without requiring additional specific operating parameter data. The actions taken by control circuit 80 in selecting operating parameters in response to a detected lead type, e.g., relating to cardiac oversensing detection or any other operating parameters selected in response to a detected lead type, may be "known" by external device 50. External device 50 may generate a GUI that corresponds to the detected lead type with automatically selected operating parameters based on the notification of lead type received from IMD 14 without requiring additional specific data, such as a notification that a far-field P-wave oversensing and/or T-wave oversensing detection is turned on or off and/or other operating parameters relating to detecting or avoiding cardiac event oversensing have been set or adjusted.

At block 256, control circuit 80 may enable a noise detection algorithm in response to detecting an integrated lead type. In some examples, a noise detection algorithm may be disabled by default and remain disabled in response to detecting a true bipolar lead but may be programmable on or off by a user interacting with external device 50. Non-cardiac myopotential noise and EMI may be more likely to be present in a tip-to-coil signal sensed by an integrated bipolar lead than in a tip-to-ring signal sensed by a true bipolar lead due to the larger surface area of the elongated coil electrode compared to the ring electrode. As such, control circuit 80 may be configured to always enable noise detection at block 256 in response to integrated bipolar lead detection.

In various examples, at block 256, control circuit 80 may select a noise detection algorithm, signal features determined and analyzed for detecting non-cardiac signal noise, and/or thresholds, ranges, or other values or criteria applied to sensed cardiac signal features determined for noise detection that are different than the noise detection noise detection operating parameters that are selected at block 208 of FIG. 6 in response to detecting a true bipolar lead. Any of a variety of noise detection algorithms may be implemented in IMD 14 in conjunction with the techniques disclosed herein. Various signal features that may be determined from a bipolar sensed signal for detecting non-cardiac noise may include signal pulse counts, mean rectified amplitude, normalized mean rectified amplitude, mean period, spectral width, and low slope content, as examples. Control circuit 80 may select different signal features and/or different thresholds or other criteria applied to the signal features for detecting non-cardiac noise contamination of the sensed signal depending on the detected lead type. Example noise detection methods are generally disclosed in the above-incorporated U.S. Patent No. 7,774,049 (Ghanem, et al.) and in U.S. Patent No. 7,783,354 (Gunderson).

Selection of operating parameters for controlling non-cardiac noise detection at block 256 may include selecting report parameters used in generating GUI(s) for communicating to a clinician or other user selected and/or programmable parameters relating to noise detection, data acquired relating to noise detection, data labeling, or the like. Control circuit 80 may transmit report parameters pertaining to selected noise detection operating parameters, or, as described above, external device 50 may receive a detected lead type notification from IMD 14 and respond accordingly in generating GUIs that include reports, data, data labeling etc. that correspond to known operating parameter selections made by control circuit 80 in response to the detected lead type. External device processor 52 may control display unit 54 to display a GUI that includes a window, table menu, icon or other reports of automatically selected and/or recommended noise detection control parameter settings, which may be programmable or non-programmable by a user interacting with the GUI.

At block 258, control circuit 80 may select operating parameters used by processing circuitry in determining whether an unknown sensed event waveform morphology matches a cardiac event signal template. During oversensing detection and/or tachyarrhythmia detection algorithms, IMD signal processing circuitry included in sensing circuit 86 and/or control circuit 80 may determine a morphology matching score between one or more unknown signal waveforms of the sensed cardiac electrical signal and an established cardiac event morphology template, e.g., an R-wave template. Sensed waveforms having a high morphology matching score may be determined to be true R-waves, which in some instances may be a premature ventricular contraction (PVC), as opposed to being an oversensed cardiac event signal, oversensed noise or an aberrantly conducted depolarization signal, e.g., a non-sinus tachycardia R-wave, or fibrillation wave.

The cardiac event morphology template may be established by collecting a predetermined number of sensed cardiac signal waveforms that are determined to have high cross-correlation with each other and averaging the waveforms to obtain a template that can be stored in memory 82. In some instances, an integrated bipolar lead may be replacing a true bipolar lead when the medical lead type is detected. In this case, a cardiac event template may be stored in memory 82 that was established based on the bipolar signal sensed using the true bipolar lead. Control circuit 80 may respond to an integrated bipolar lead detection by establishing a new cardiac event morphology template from the bipolar tip-to-coil signal sensed by the integrated bipolar lead. In other instances, detection of a true bipolar lead may be a change from a previously connected integrated bipolar lead, requiring a new cardiac event morphology template to be established at block 208 of FIG. 6.

Other morphology matching operating parameters that may be selected at block 258 in response to detecting an integrated bipolar lead (or at block 208 of FIG. 6 when a true bipolar lead is detected) include a morphology match threshold. When the signal processing circuitry determines that a morphology matching score is greater than the match threshold, the sensed signal is determined to be a true cardiac event corresponding to the established template, e.g., a true R-wave. The morphology match threshold may be set differently by control circuit 80 when an integrated bipolar lead is detected compared to when a true bipolar lead is detected. The variability in normal QRS waveform morphology (and in PVC waveform morphologies) may be greater in the integrated bipolar lead signal than in the true bipolar lead signal. As such, a lower morphology match threshold may be applied to morphology matching scores when an integrated bipolar lead is detected. A relatively higher morphology match threshold may be selected at block 204 of FIG. 6 when a true bipolar lead is detected.

The various operating parameters and report parameters that are described in conjunction with FIG. 7 and in reference to FIG. 6 are illustrative in nature and not intended to be limiting or exhaustive. Some or all of the example operating parameters described above may be selected by control circuit 80 in any combination based on the detected lead type. The selection by control circuit 80 of some of the example operating parameters described above for use by IMD signal processing circuitry for determining a need for electrical stimulation therapy may be omitted or excluded. Furthermore, control circuit 80 may select other operating parameters, alone or in combination with any of the examples given above, for use by signal processing circuitry for determining a heart rhythm and/or determining a need for electrical stimulation therapy. After selecting one or any combination of operating parameters, control circuit 80, in cooperation with sensing circuit 86 and therapy delivery circuit 84, may operate according to the selected operating parameter(s) for sensing cardiac signals, determining a need for electrical stimulation therapy, and delivering electrical stimulation therapy.

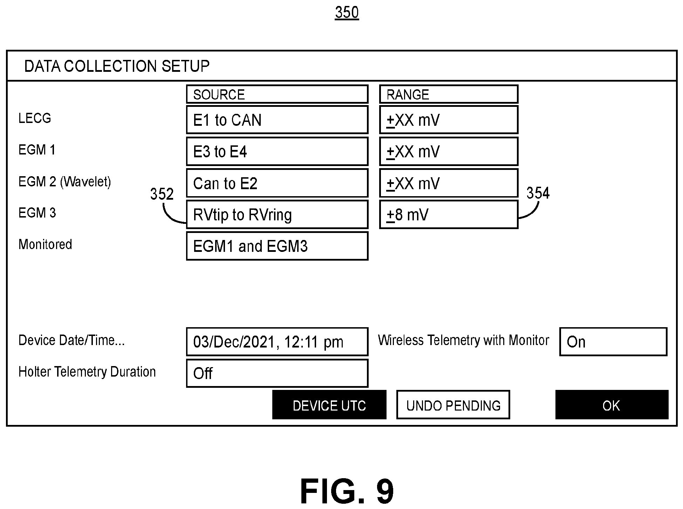

FIG. 8 is a diagram of a GUI 300 that may be displayed by external device 50 in response to receiving data transmitted from IMD 14 according to one example. The GUI 300 reports various control parameters used by IMD 14 for pacing and sensing operations. The control parameters in GUI 300 may be programmable by a user and/or set to default values and/or set to values selected by IMD control circuit 80 in response to a detected lead type. In the example shown in FIG. 8 and in other diagrams of GUIs shown in FIGs. 9-11, it is to be understood that any control parameter values or settings shown are merely illustrative in nature and are not intended to be limiting with regard to a possible value or range of values that may be set automatically by IMD 14 or programmable by a user. Furthermore, various control parameters and associated values that may be displayed in a GUI may be specific to an IMD system and may variously relate to any of pacing mode, pacing lower rate, AV intervals, blanking intervals, pacing pulse amplitude, pulse width, sensitivity, sensing electrode vectors, pacing electrode vectors, impedance measurement vectors or any other pacing, sensing, arrhythmia detection, therapy delivery or other pacemaker function or pacemaker feature control parameters or settings. At least some of the parameters and/or associated values displayed in a GUI may be patient-specific and/or device specific. The control parameters and values displayed in a GUI can therefore vary between patients and between IMD systems in which techniques disclosed herein are being implemented.

In the example shown, IMD 14 is configured to deliver cardiac pacing in a dual chamber pacing mode (e.g., a DDD pacing mode) in which both atrial and RV pacing and sensing are programmed to be "bipolar." In this example, the RA lead coupled to IMD 14 may be a bipolar lead having at an RA tip and an RA ring electrode, as shown in the examples of FIGs. 1 and 2. When the IMD housing 15 can be used as an active can electrode for delivering CV/DF shocks, the only pace and sense polarity available may be bipolar from the true bipolar RA lead 16. As such, the atrial pace polarity and the atrial sense polarity (collectively 301) are each displayed as being "bipolar" and are not programmable settings because the IMD housing 15 is unavailable for use as a return electrode for unipolar sensing and pacing.

The RV pace polarity and the RV sense polarity may be "bipolar" as indicated in display windows 302 and 304. However, each of the pace polarity and the sense polarity display windows 302 and 304 may further indicate if the bipolar electrode vector is a tip-to-coil bipolar pacing or sensing vector (as shown) or a tip-to-ring bipolar pacing or sensing vector based on a determined lead type. In response to receiving a notification transmitted from IMD 14 indicating an integrated bipolar lead is detected, external device processor 52 may generate the data for display in GUI 300 on display unit 54 including the pace polarity window 302 and the sense polarity window 304 indicating "bipolar tip-to-coil" as shown. The pace polarity window 302 and the sense polarity window 304 may be set to the fixed parameter values of bipolar tip-to-coil displayed without a user-programmable option because the ring electrode is not available for bipolar tip-to-ring pacing or sensing when an integrated bipolar lead is coupled to IMD 14. Accordingly, windows 302 and 304 may be grayed out or fixed windows that disable user programming of the pace and sense polarities when an integrated bipolar lead is detected and the "tip-to-coil" vector can be displayed.

When a true bipolar lead is detected, external device 50 may receive a notification transmitted from IMD 14 indicating that a true bipolar lead is detected. IMD 14 may transmit a notification that the pace and sense polarities are selected to be bipolar tip-to-ring and a notification that the pace and sense polarities are programmable to be bipolar tip-to-ring or bipolar tip-to-coil because both the RV ring electrode and the RV coil electrode are present on the true bipolar lead and may be available for bipolar pacing and sensing. Alternatively, external device processor 52 may be configured to generate GUI 300 for display by display unit 54 including the windows 302 and 304 set to the reporting parameter of bipolar tip-to-ring for both pace and sense polarities in response to receiving the true bipolar lead detection notification from IMD 14 without requiring additional data or notifications relating to the pace and sense polarities. GUI 300 may display "Bipolar tip-to-ring" (or similar user notification), and the windows 302 and 304 may be pull down windows that allow a user to select bipolar tip-to-ring or bipolar tip-to-coil as a programmable bipolar electrode vector for sensing or pacing when a true bipolar lead is detected. In other examples, the pace polarity and/or the sense polarity may be fixed as a bipolar tip-to-ring polarity without the option of user programmability to the tip-to-coil electrode vector when a true bipolar lead is detected by IMD 14.

GUI 300 may include windows reporting other operating parameters that can be selected by IMD 14 based on the detected lead type. For example, the post-sense and/or post-pace ventricular blanking period, which may be displayed in a pop-up window, for example, when a user selects window 306. The post-sense and/or post-pace blanking period may be set to a longer blanking period by IMD control circuit 80 when the detected lead type is integrated bipolar. The GUI 300 may display the blanking period set by IMD control circuit 80 based on the detected lead type, which may be 30 to 450 ms, or 50 to 150 ms as examples. The post-ventricular blanking period that is applied following a sensed event signal may be fixed, e.g., 50 ms for a true bipolar lead and 80 ms for an integrated bipolar lead. In other examples, post-ventricular blanking period may be programmable. The GUI 300 may include a drop down menu of programmable blanking periods available for the detected lead type in window 306. The shortest available blanking period that is user programmable may be longer when an integrated bipolar lead is detected than when a true bipolar lead is detected. For instance, the blanking period may be as short as 50 ms for a true bipolar lead, but the minimum programmable blanking period for an integrated bipolar lead may be 75 ms, as examples with no limitation intended.

When IMD 14 is a multi-chamber device including LV lead 17 (as shown in FIG. 1), control circuit 80 may select a post-ventricular blanking period applied to avoid double-sensing of the same R-wave and a post-pace ventricular blanking period applied to the other ventricular sensing channel when a pacing pulse is delivered in a first ventricular chamber. The post-ventricular blanking period following a sensed R-wave in the RV may be different than the post-pace ventricular blanking period applied to the sensed RV signal following an LV pacing pulse (to avoid cross-chamber sensing of the LV pacing pulse as an R-wave in the RV). Accordingly, one or more blanking periods may be selected by control circuit 80, displayed in GUI 300, and applied to the bipolar sensed signal based on the detected lead type.

FIG. 9 is a diagram of a data collection setup GUI 350 that may be displayed by external device 50 including one or more reported operating parameters or data based on the lead type detected by IMD 14. In this example, the sensing electrode vectors that are selected for sensing cardiac electrical signals and for processing and analysis by IMD signal processing circuitry may be reported. As shown, one or more EGM signals may be listed in the data collection setup GUI 350 with a corresponding indication of the sensing electrode vector and amplitude range (in millivolts) of an ADC included in the respective sensing channel of sensing circuit 86.

A bipolar sensed EGM signal, e.g., listed as EGM 3 in GUI 350, may be labeled according to the lead type detected by IMD 14 as being either a "tip-to-ring" or "tip-to-coil" source. In the examples shown, a true bipolar lead detection notification has been received by external device 50 such that the EGM 3 signal source 352 is indicated as being the bipolar RV tip-to-ring sensing electrode vector. When an integrated bipolar lead detection notification transmitted by IMD 14 is received by external device 50, GUI 350 may include a display of "RVtip to RVcoil" as the source of an EGM signal that is analyzed by signal processing circuitry of IMD 14.

The ADC range 354 for the bipolar EGM signal source may be automatically selected by control circuit 80 based on the detected lead type. The selected ADC range 354 may be transmitted to external device 50 or automatically known by external device 50 in response to receiving a notification of the detected lead type from IMD 14. For example, the ADC range may be set to be between ± 10 to ±14 Volts for a detected integrated bipolar lead. In some examples, the ADC range may be set slightly lower to be between ± 7 to ± 10 Volts for a detected true bipolar lead.

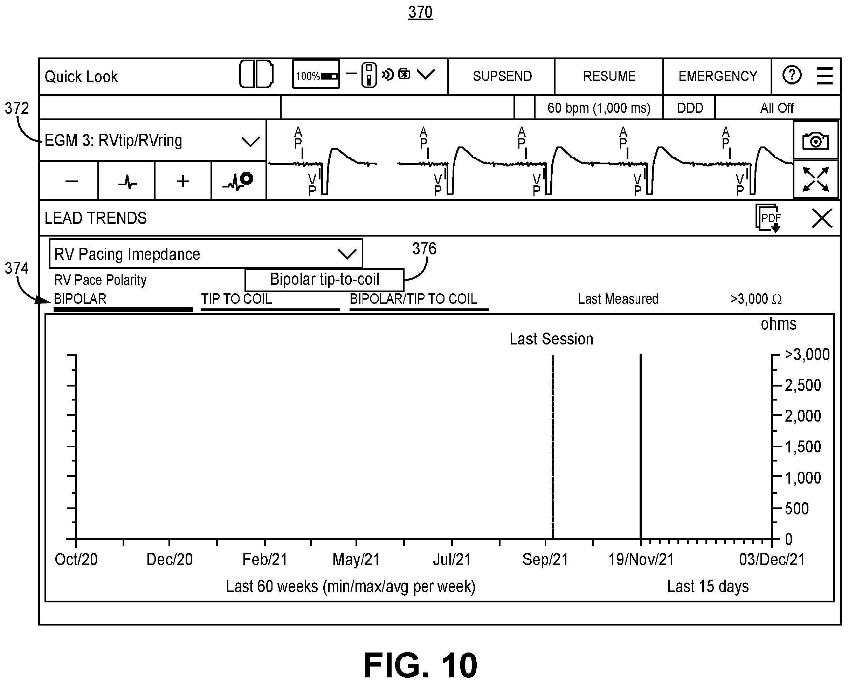

FIG. 10 is a diagram of a GUI 370 that may be displayed by external device 50 that may include data and information based on the detected lead type according to another example. The GUI 370 is shown as a report that includes a display 372 of an EGM signal received from IMD 14 via wireless telemetry. In this example, the EGM signal displayed is shown as the bipolar RVtip to RVring EGM signal. External device 50 may set the EGM signal label according to the EGM signal source determined based on a medical lead type notification received from IMD 14. In this example, a true bipolar lead determination is made by IMD 14 and a notification of this determination may be transmitted to external device 50. GUI 370 may be generated to include the appropriate "tip-to-ring" label in response to receiving the true bipolar lead determination notification. When an integrated bipolar lead determination notification is received from IMD 14, GUI 370 may be generated to include the appropriate "tip-to-coil" label of the displayed EGM signal. Displaying only an "EGM" label, a "bipolar" signal label, or "tip-to-ring" label (which may correspond to electrode terminals connected to the RV sensing channel 89 when in fact the tip-to-coil signal is being sensed) can be misleading and confusing to a user. Accordingly, the correct "tip-to-ring" or "tip-to-coil" label may be displayed to indicate the bipolar sensing vector based on the detected lead type.

The GUI 370 is further shown to include a graphical display reporting RV pacing impedance measurements. When the determined lead type is true bipolar, the RV pacing impedance measurement report may include a graphical or tabular history of bipolar tip-to-ring and bipolar tip-to-coil impedance measurements acquired by impedance measurement circuit 90 and stored in memory 82 for transmission to external device 50. A user may select on the labels 372 to toggle between a graphical display of impedance tip-to-ring and tip-to-coil impedance measurements over time. The currently selected RV pacing polarity is shown to be selected as the bipolar tip-to-coil polarity. A user may reprogram the RV pacing polarity to RV tip-to-ring by selecting the pace polarity window 376 when the determined lead type is true bipolar.

When the determined lead type notification is an integrated bipolar lead, external device 50 may adjust the report parameters in GUI 370 to display the RV pace polarity as being bipolar tip-to-coil without a user selectable option of pacing polarity. RV pacing impedance reporting of only the bipolar tip-to-coil impedance may be displayed with reporting of bipolar tip-to-ring impedance reporting unavailable or excluded. In this way, the external device modifies the GUI 370 to provide the most relevant labels and data based on the lead type notification received from IMD 14.

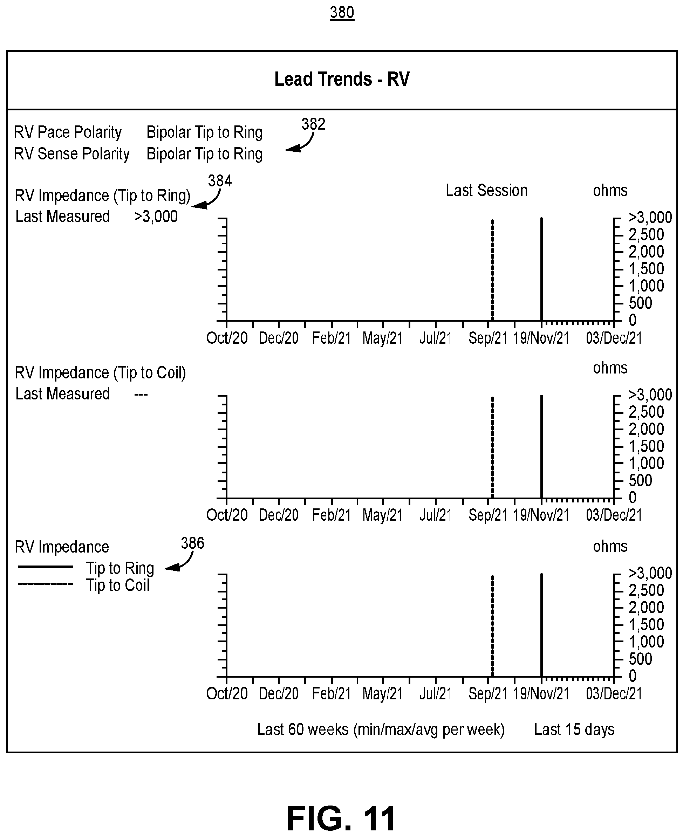

FIG. 11 is another example of a GUI 380 that includes a report of RV lead impedance measurements that may be generated by external device (or a remote patent management system) according to a lead type notification from IMD 14. As described above, external device 50 may modify the reported data and/or data labels based on a lead type notification received from IMD 14. For example, the RV pace polarity and the RV sense polarity displayed in window 382 may be labeled as "bipolar tip-to-ring" or "bipolar tip-to-coil" based on whether the determined lead type is true bipolar or integrated bipolar, respectively. An RV pace and/or RV sense polarity labeled only "bipolar" can be confusing or misleading to a clinician or other user without the additional designation of the actual bipolar electrode vector being indicated as either tip-to-ring or tip-to-coil, particularly when taken into account with pacing capture thresholds, lead impedance measurements or other lead and electrode vector related data.

The graphical report of RV impedances may be tailored according to a received medical lead type determination notification. When an integrated bipolar lead is detected by IMD 14, external device 50 (or a remote patient management system) may generate data for display in GUI that excludes "tip-to-ring" impedance measurements 384 and 386 and specifies all impedance measurements as "tip-to-coil" impedance measurements. In the example shown, a true bipolar lead type is detected such that the impedance report includes reporting of RV tip-to-ring impedances and associated labeling 384 and 386 in addition to bipolar tip-to-coil impedance measurements.

In GUI 380 and other GUIs shown and described herein, a pace or sense polarity and/or impedance measurements that are reported only as being "bipolar" may be confusing to a clinician or other user. For example, reported impedances and/or capture thresholds for a bipolar tip-to-ring pacing electrode configuration may fall into a different "normal" range than the bipolar tip-to-coil pacing electrode configuration when an integrated bipolar lead is connected to IMD 14. Accordingly, specifying the bipolar polarity for pacing and sensing polarities as being either "tip-to-ring" or "tip-to-coil" may reduce the likelihood of user confusion, unnecessary troubleshooting, or improper programming of IMD 14.

Furthermore, including both "tip-to-ring" and "tip-to-coil" reporting and labeling, e.g., in the RV impedance lead trends report shown in GUI 380, may be misleading or confusing to a user when the IMD 14 is connected to an integrated bipolar lead. The tip-to-ring and tip-to-coil impedance measurements may be equal or the tip-to-ring impedance measurements may be omitted according to operating parameters set by IMD control circuit 80 when an integrated bipolar lead is detected by IMD 14. As such, the detected lead type notification enables external device 50 to modify reports and data labeling to minimize user confusion and present the most relevant data and information based on the type of lead that is connected to IMD 14.

Accordingly, the techniques set forth herein provide specific improvements to the computer-related field of programming medical devices and reporting medical device-related information and data that have practical applications. For example, the use of the techniques herein may enable external device 50 to generate visualizations of cardiac electrical signal data, impedance data, pacing and sensing electrode vector selections, and other operating parameter selections and programmability relevant to the type of lead connected to IMD 14. Such visualizations may more accurately inform a clinician or user as to how IMD 14 is expected to perform and provide the most relevant reports and programmable parameter settings and recommendations to reduce the likelihood of human error in programming IMD operating parameters. Furthermore, the techniques disclosed herein may reduce the complexity of programming IMD 14. As such, the techniques disclosed herein may enable a medical device, such as IMD 14, to be programmed to process and analyze cardiac electrical signals according to operating parameters in a manner that is simplified, flexible, and patient- and lead-specific such that the IMD may reliably sense cardiac event signals, determine heart rhythms, and determine a need for electrical stimulation therapy.

It should be understood that, depending on the example, certain acts or events of any of the methods described herein can be performed in a different sequence, may be added, merged, or left out altogether (e.g., not all described acts or events are necessary for the practice of the method). Moreover, in certain examples, acts or events may be performed concurrently, e.g., through multi-threaded processing, interrupt processing, or multiple processors, rather than sequentially. In addition, while certain aspects of this disclosure are described as being performed by a single circuit or unit for purposes of clarity, it should be understood that the techniques of this disclosure may be performed by a combination of units or circuits associated with, for example, a medical device.

In one or more examples, the functions described may be implemented in hardware, software, firmware, or any combination thereof. If implemented in software, the functions may be stored as one or more instructions or code on a computer-readable medium and executed by a hardware-based processing unit. Computer-readable media may include computer-readable storage media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPLAs), or other equivalent integrated or discrete logic circuitry. Accordingly, the term "processor," as used herein may refer to any of the foregoing structure or any other structure suitable for implementation of the techniques described herein. Also, the techniques could be fully implemented in one or more circuits or logic elements.

Thus, a medical device has been presented in the foregoing description with reference to specific examples. It is to be understood that various aspects disclosed herein may be combined in different combinations than the specific combinations presented in the accompanying drawings. It is appreciated that various modifications to the referenced examples may be made without departing from the scope of the disclosure

## Claims

1. A medical device (14), comprising:
a connector bore (150) configured to receive a proximal portion of a medical lead (60), the connector bore (150) comprising at least a first electrical contact (160) and a second electrical contact (162);
an impedance measurement circuit (90) configured to obtain an impedance measurement between a first electrode terminal corresponding to the first electrical contact (160) of the connector bore (150) and a second electrode terminal corresponding to the second electrical contact (162) of the connector bore (150); and
a control circuit (80) configured to:
determine a medical lead type of the medical lead (60) received by the connector bore (150) based on the impedance measurement, wherein the medical lead type is determined to be one of an integrated bipolar lead or a true bipolar lead; and
select at least one operating parameter setting based on the determined medical lead type; and
signal processing circuitry (86) configured to receive a cardiac electrical signal and process the cardiac electrical signal according to the at least one operating parameter setting for determining a need for an electrical stimulation therapy; and
a therapy delivery circuit (84) configured to deliver the electrical stimulation therapy in response to the signal processing circuitry (86) determining the need for the electrical stimulation therapy.

2. The medical device (14) of claim 1, wherein:
the signal processing circuitry (86) includes a notch filter; and
the control circuit (80) is further configured to select the at least one operating parameter setting by enabling the notch filter in response to determining the medical lead type as being the integrated bipolar lead.

3. The medical device (14) of any of claims 1-2, wherein:
the signal processing circuitry (86) is configured to detect cardiac event oversensing; and
the control circuit (80) is further configured to select the at least one operating parameter setting by one of:
enabling the cardiac event oversensing by the signal processing circuitry (86) in response to determining the medical lead type as being the integrated bipolar lead; or
disabling the cardiac event oversensing by the signal processing circuitry (86) in response to determining the medical lead type as being the true bipolar lead.

4. The medical device (14) of claim 3, wherein the signal processing circuitry (86) is further configured to detect the cardiac event oversensing by detecting P-wave oversensing.

5. The medical device (14) of any of claims 1-4, wherein:
the signal processing circuitry (86) is configured to detect cardiac event oversensing by:
sensing a plurality of cardiac event signals from the cardiac electrical signal;
determining at least one signal feature from each of the plurality of cardiac event signals; and
determining an alternating pattern of the determined signal features based on at least one threshold applied to the determined signal features; and
the control circuit (80) is further configured to select the at least one operating parameter setting by one of:
selecting a first value of the threshold in response to determining the medical lead type being the integrated bipolar lead; or
selecting a second value of the threshold different than the first value in response to determining the medical lead type being the true bipolar lead.

6. The medical device (14) of any of claims 1-5, wherein:
the signal processing circuitry (86) is configured to detect cardiac event oversensing by:
sensing a plurality of cardiac event signals from the cardiac electrical signal;
determining at least one signal feature from each of the plurality of cardiac event signals; and
determining an alternating pattern of the determined signal features based on at least one threshold applied to the determined signal features; and
the control circuit (80) is further configured to select the at least one operating parameter setting by one of:
selecting a first signal feature to be determined by the signal processing circuitry (86) from each of the plurality of cardiac event signals in response to determining the medical lead type being the integrated bipolar lead; or
selecting a second signal feature to be determined by the signal processing circuitry (86) from each of the plurality of cardiac event signals in response to determining the medical lead type being the true bipolar lead, the second signal feature being different than the first signal feature.

7. The medical device (14) of any of claims 1-6, wherein:
the signal processing circuitry (86) is further configured to detect non-cardiac noise signals in the cardiac electrical signal; and
the control circuit (80) is further configured to select the at least one operating parameter setting by enabling detecting non-cardiac noise signals in the cardiac electrical signal by the signal processing circuitry (86) in response to determining the medical lead type being the integrated bipolar lead.

8. The medical device (14) of any of claims 1-7, wherein:
the signal processing circuitry (86) is further configured to:
determine a morphology matching score between a previously established morphology template corresponding to a cardiac event signal and an unknown event signal in the cardiac electrical signal; and
compare the morphology matching score to a morphology match threshold for determining when the unknown signal is the cardiac event signal; and
the control circuit (80) is further configured to select the at least one operating parameter setting by one of:
selecting a first value of the morphology match threshold in response to determining the medical lead type being the integrated bipolar lead; or
selecting a second value of the morphology match threshold different than the first value in response to determining the medical lead type being the true bipolar lead.

9. The medical device (14) of any of claims 1-8, wherein:
the signal processing circuitry (86) is further configured to:
determine a morphology matching score between a previously established morphology template corresponding to a cardiac event signal and an unknown event signal in the cardiac electrical signal; and
compare the morphology matching score to a morphology match threshold for determining when the unknown event signal is the cardiac event signal; and
the control circuit (80) is further configured to select the at least one operating parameter setting by:
determining that the medical lead type is different than a previously determined medical lead type; and
re-establishing the morphology template from the cardiac electrical signal in response to determining that the medical lead type is different than the previously determined medical lead type.

10. The medical device (14) of any of claims 1-9, further comprising a memory, wherein:
the impedance measurement circuit (90) is further configured to determine a bipolar lead impedance measurement; and
the control circuit (80) is further configured to:
select the at least one operating parameter setting by one of:
selecting a first range of a normal bipolar lead impedance range in response to determining the medical lead type being the integrated bipolar lead; or
selecting a second range of the normal bipolar lead impedance range in response to determining the medical lead type being the true bipolar lead, where the second range is different than the first range;
determine that the bipolar lead impedance measurement is outside the normal bipolar lead impedance range; and
generate an output in response to determining that the bipolar lead impedance measurement is outside the normal bipolar lead impedance range; and
the memory being configured to store the bipolar lead impedance measurement with the output generated by the control circuit (80).

11. The medical device (14) of any of claims 1-10, further comprising a telemetry circuit configured to transmit data signals:
wherein the control circuit (80) is further configured to set at least one report parameter based on the determined lead type; and
the telemetry circuit is configured to transmit the report parameter to another medical device (14) for use in generating a display of a graphical user interface.

12. The medical device (14) of any of claims 1-11, wherein the control circuit (80) is further configured to determine the medical lead type by:
comparing the impedance measurement to a threshold impedance;
responsive to the impedance measurement being less than the threshold impedance, determining the medical lead type as being the integrated bipolar lead having a first elongated coil electrode of the integrated bipolar lead electrically coupled to the first electrode terminal and electrically coupled to the second electrode terminal; or
responsive to the impedance measurement being greater than the threshold impedance, determining the medical lead type as being the true bipolar lead having a second elongated coil electrode of the true bipolar lead electrically coupled to the first electrode terminal and a ring electrode of the true bipolar lead electrical coupled to the second electrode terminal.

13. A non-transitory, computer readable medium storing a set of instructions that, when executed by a control circuit (80) of a medical device (14), cause the medical device (14) to:
obtain an impedance measurement between a first electrode terminal corresponding to a first electrical contact (160) of a connector bore (150) of the medical device (14) and a second electrode terminal corresponding to a second electrical contact (162) of the connector bore (150), the connector bore (150) being configured to receive a proximal portion of a medical lead (60);
based on the impedance measurement, determine a medical lead type of the medical lead received by the connector bore (150), wherein the medical lead type is determined to be one of an integrated bipolar lead or a true bipolar lead;
select at least one operating parameter setting based on the determined medical lead type;
process a cardiac electrical signal according to the at least one operating parameter setting for determining a need for an electrical stimulation therapy; and
deliver the electrical stimulation therapy in response to determining the need for the electrical stimulation therapy.

## Patentansprüche

1. Medizinische Vorrichtung (14), die Folgendes umfasst:
eine Verbinderbohrung (150), die dazu konfiguriert ist, einen proximalen Teil einer medizinischen Leitung (60) aufzunehmen, wobei die Verbinderbohrung (150) wenigstens einen ersten elektrischen Kontakt (160) und einen zweiten elektrischen Kontakt (162) umfasst;
einen Impedanzmessungsschaltkreis (90), der dazu konfiguriert ist, eine Impedanzmessung zwischen einem ersten Elektrodenanschluss, der dem ersten elektrischen Kontakt (160) der Verbinderbohrung (150) entspricht, und einem zweiten Elektrodenanschluss, der dem zweiten elektrischen Kontakt (162) der Verbinderbohrung (150) entspricht, zu erhalten; und
einen Steuerschaltkreis (80), der zu Folgendem konfiguriert ist:
Bestimmen eines medizinischen Leitungstyps der medizinischen Leitung (60), die durch die Verbinderbohrung (150) aufgenommen wird, basierend auf der Impedanzmessung, wobei bestimmt wird, dass der medizinische Leitungstyp eine einer integriert bipolaren Leitung oder einer echt bipolaren Leitung ist; und
Auswählen wenigstens einer Betriebsparametereinstellung basierend auf dem bestimmten medizinischen Leitungstyp; und
eine Signalverarbeitungsschaltungsanordnung (86), die dazu konfiguriert ist, ein elektrisches Herzsignal zu empfangen und das elektrische Herzsignal gemäß der wenigstens einen Betriebsparametereinstellung zum Bestimmen eines Bedarfs an einer elektrischen Stimulationstherapie zu verarbeiten; und
ein Therapiebereitstellungsschaltkreis (84), der dazu konfiguriert ist, die elektrische Stimulationstherapie als Reaktion darauf bereitzustellen, dass die Signalverarbeitungsschaltungsanordnung (86) den Bedarf an der elektrischen Stimulationstherapie bestimmt.

2. Medizinische Vorrichtung (14) nach Anspruch 1, wobei:
die Signalverarbeitungsschaltungsanordnung (86) ein Kerbfilter beinhaltet; und
der Steuerschaltkreis (80) ferner dazu konfiguriert ist, die wenigstens eine Betriebsparametereinstellung durch Aktivieren des Kerbfilters als Reaktion auf das Bestimmen davon auszuwählen, dass der medizinische Leitungstyp die integriert bipolare Leitung ist.

3. Medizinische Vorrichtung (14) nach einem der Ansprüche 1-2, wobei:
die Signalverarbeitungsschaltungsanordnung (86) dazu konfiguriert ist, eine Herzereignisübererfassung zu detektieren; und
der Steuerschaltkreis (80) ferner dazu konfiguriert ist, die wenigstens eine Betriebsparametereinstellung durch eines von Folgendem auszuwählen:
Aktivieren der Herzereignisübererfassung durch die Signalverarbeitungsschaltungsanordnung (86) als Reaktion auf das Bestimmen davon, dass der medizinische Leitungstyp die integriert bipolare Leitung ist; oder
Deaktivieren der Herzereignisübererfassung durch die Signalverarbeitungsschaltungsanordnung (86) als Reaktion auf das Bestimmen davon, dass der medizinische Leitungstyp die echt bipolare Leitung ist.

4. Medizinische Vorrichtung (14) nach Anspruch 3, wobei die Signalverarbeitungsschaltungsanordnung (86) ferner dazu konfiguriert ist, die Herzereignisübererfassung durch Detektieren einer P-Wellen-Übererfassung zu detektieren.

5. Medizinische Vorrichtung (14) nach einem der Ansprüche 1-4, wobei:
die Signalverarbeitungsschaltungsanordnung (86) dazu konfiguriert ist, eine Herzereignisübererfassung durch Folgendes zu detektieren:
Erfassen mehrerer Herzereignissignale aus dem elektrischen Herzsignal;
Bestimmen wenigstens eines Signalmerkmals aus jedem der mehreren Herzereignissignale; und
Bestimmen eines alternierenden Musters der bestimmten Signalmerkmale basierend auf wenigstens einer Schwelle, die auf die bestimmten Signalmerkmale angewendet wird; und
der Steuerschaltkreis (80) ferner dazu konfiguriert ist, die wenigstens eine Betriebsparametereinstellung durch eines von Folgendem auszuwählen:
Auswählen eines ersten Wertes der Schwelle als Reaktion auf das Bestimmen davon, dass der medizinische Leitungstyp die integriert bipolare Leitung ist; oder
Auswählen eines zweiten Wertes der Schwelle, der sich von dem ersten Wert unterscheidet, als Reaktion auf das Bestimmen davon, dass der medizinische Leitungstyp die echt bipolare Leitung ist.

6. Medizinische Vorrichtung (14) nach einem der Ansprüche 1-5, wobei:
die Signalverarbeitungsschaltungsanordnung (86) dazu konfiguriert ist, eine Herzereignisübererfassung durch Folgendes zu detektieren:
Erfassen mehrerer Herzereignissignale aus dem elektrischen Herzsignal;
Bestimmen wenigstens eines Signalmerkmals aus jedem der mehreren Herzereignissignale; und
Bestimmen eines alternierenden Musters der bestimmten Signalmerkmale basierend auf wenigstens einer Schwelle, die auf die bestimmten Signalmerkmale angewendet wird; und
der Steuerschaltkreis (80) ferner dazu konfiguriert ist, die wenigstens eine Betriebsparametereinstellung durch eines von Folgendem auszuwählen:
Auswählen eines ersten Signalmerkmals, das durch die Signalverarbeitungsschaltungsanordnung (86) zu bestimmen ist, aus jedem der mehreren Herzereignissignale als Reaktion darauf, dass bestimmt wird, dass der medizinische Leitungstyp die integriert bipolare Leitung ist; oder
Auswählen eines zweiten Signalmerkmals, das durch die Signalverarbeitungsschaltungsanordnung (86) zu bestimmen ist, aus jedem der mehreren Herzereignissignale als Reaktion auf das Bestimmen davon, dass der medizinische Leitungstyp die echt bipolare Leitung ist, wobei sich das zweite Signalmerkmal von dem ersten Signalmerkmal unterscheidet.

7. Medizinische Vorrichtung (14) nach einem der Ansprüche 1-6, wobei:
die Signalverarbeitungsschaltungsanordnung (86) ferner dazu konfiguriert ist, nicht herzbezogene Rauschsignale in dem elektrischen Herzsignal zu detektieren; und
der Steuerschalkreis (80) ferner dazu konfiguriert ist, die wenigstens eine Betriebsparametereinstellung auszuwählen, indem das Detektieren von nichtherzbezogenen Rauschsignalen in dem elektrischen Herzsignal durch die Signalverarbeitungsschaltungsanordnung (86) als Reaktion auf das Bestimmen davon, dass der medizinische Leitungstyp die integriert bipolare Leitung ist, ermöglicht wird.

8. Medizinische Vorrichtung (14) nach einem der Ansprüche 1-7, wobei:
die Signalverarbeitungsschaltungsanordnung (86) ferner zu Folgendem konfiguriert ist:
Bestimmen einer Morphologieübereinstimmungsbewertung zwischen einer zuvor eingerichteten Morphologievorlage, die einem Herzereignissignal entspricht, und einem unbekannten Ereignissignal in dem elektrischen Herzsignal; und
Vergleichen der Morphologieübereinstimmungsbewertung mit einer Morphologieübereinstimmungsschwelle zum Bestimmen davon, ob das unbekannte Signal das Herzereignissignal ist; und
der Steuerschaltkreis (80) ferner dazu konfiguriert ist, die wenigstens eine Betriebsparametereinstellung durch eines von Folgendem auszuwählen:
Auswählen eines ersten Wertes der Morphologieübereinstimmungsschwelle als Reaktion auf das Bestimmen davon, dass der medizinische Leitungstyp die integriert bipolare Leitung ist; oder
Auswählen eines zweiten Wertes der Morphologieübereinstimmungsschwelle, der sich von dem ersten Wert unterscheidet, als Reaktion auf das Bestimmen davon, dass der medizinische Leitungstyp die echte bipolare Leitung ist.

9. Medizinische Vorrichtung (14) nach einem der Ansprüche 1-8, wobei:
die Signalverarbeitungsschaltungsanordnung (86) ferner zu Folgendem konfiguriert ist:
Bestimmen einer Morphologieübereinstimmungsbewertung zwischen einer zuvor eingerichteten Morphologievorlage, die einem Herzereignissignal entspricht, und einem unbekannten Ereignissignal in dem elektrischen Herzsignal; und
Vergleichen der Morphologieübereinstimmungsbewertung mit einer Morphologieübereinstimmungsschwelle zum Bestimmen davon, ob das unbekannte Ereignissignal das Herzereignissignal ist; und
der Steuerschaltkreis (80) ferner dazu konfiguriert ist, die wenigstens eine Betriebsparametereinstellung durch Folgendes auszuwählen:
Bestimmen, dass sich der medizinische Leitungstyp von einem zuvor bestimmten medizinischen Leitungstyp unterscheidet; und
erneutes Einrichten der Morphologievorlage aus dem elektrischen Herzsignal als Reaktion auf das Bestimmen davon, dass sich der medizinische Leitungstyp von dem zuvor bestimmten medizinischen Leitungstyp unterscheidet.

10. Medizinische Vorrichtung (14) nach einem der Ansprüche 1-9, die ferner einen Speicher umfasst, wobei:
der Impedanzmessungsschaltkreis (90) ferner dazu konfiguriert ist, eine Bipolarleitungsimpedanzmessung zu bestimmen; und
der Steuerschalkreis (80) ferner zu Folgendem konfiguriert ist: Auswählen der wenigstens einen Betriebsparametereinstellung durch eines von Folgendem:
Auswählen eines ersten Bereichs eines normalen Bipolarleitungsimpedanzbereichs als Reaktion auf das Bestimmen davon, dass der medizinische Leitungstyp die integriert bipolare Leitung ist; oder
Auswählen eines zweiten Bereichs des normalen Bipolarleitungsimpedanzbereichs als Reaktion auf das Bestimmen davon, dass der medizinische Leitungstyp die echt bipolare Leitung ist, wobei sich der zweite Bereich von dem ersten Bereich unterscheidet;
Bestimmen, dass die Bipolarleitungsimpedanzmessung außerhalb des normalen Bipolarleitungsimpedanzbereichs liegt; und
Erzeugen einer Ausgabe als Reaktion auf das Bestimmen davon, dass die Bipolarleitungsimpedanzmessung außerhalb des normalen Bipolarleitungsimpedanzbereichs liegt; und
wobei der Speicher dazu konfiguriert ist, die Bipolarleitungsimpedanzmessung mit der durch den Steuerschaltkreis (80) erzeugten Ausgabe zu speichern.

11. Medizinische Vorrichtung (14) nach einem der Ansprüche 1-10, die ferner einen Telemetrieschaltkreis umfasst, der dazu konfiguriert ist, Datensignale zu übertragen:
wobei der Steuerschaltkreis (80) ferner dazu konfiguriert ist, wenigstens einen Berichtparameter basierend auf dem bestimmten Leitungstyp einzustellen; und
der Telemetrieschaltkreis dazu konfiguriert ist, den Berichtparameter an eine andere medizinische Vorrichtung (14) zur Verwendung beim Erzeugen einer Anzeige einer grafischen Benutzeroberfläche zu übertragen.

12. Medizinische Vorrichtung (14) nach einem der Ansprüche 1-11, wobei der Steuerschaltkreis (80) ferner dazu konfiguriert ist, den medizinischen Leitungstyp durch Folgendes zu bestimmen:
Vergleichen der Impedanzmessung mit einer Schwellenimpedanz;
als Reaktion darauf, dass die Impedanzmessung kleiner als die Schwellenimpedanz ist, Bestimmen des medizinischen Leitungstyps als die integriert bipolare Leitung mit einer ersten länglichen Spulenelektrode der integriert bipolaren Leitung, die elektrisch mit dem ersten Elektrodenanschluss gekoppelt ist und elektrisch mit dem zweiten Elektrodenanschluss gekoppelt ist; oder
als Reaktion darauf, dass die Impedanzmessung größer als die Schwellenimpedanz ist, Bestimmen des medizinischen Leitungstyps als die echt bipolare Leitung mit einer zweiten länglichen Spulenelektrode der echt bipolaren Leitung, die elektrisch mit dem ersten Elektrodenanschluss gekoppelt ist, und einer Ringelektrode der echt bipolaren Leitung, die elektrisch mit dem zweiten Elektrodenanschluss gekoppelt ist.

13. Nichtflüchtiges computerlesbares Medium, das einen Satz von Anweisungen speichert, der bei Ausführung durch einen Steuerschaltkreis (80) einer medizinischen Vorrichtung (14) die medizinische Vorrichtung (14) zu Folgendem veranlasst:
Erhalten einer Impedanzmessung zwischen einem ersten Elektrodenanschluss, der einem ersten elektrischen Kontakt (160) einer Verbinderbohrung (150) der medizinischen Vorrichtung (14) entspricht, und einem zweiten Elektrodenanschluss, der einem zweiten elektrischen Kontakt (162) der Verbinderbohrung (150) entspricht, wobei die Verbinderbohrung (150) dazu konfiguriert ist, einen proximalen Teil einer medizinischen Leitung (60) aufzunehmen;
Bestimmen, basierend auf der Impedanzmessung, eines medizinischen Leitungstyps der medizinischen Leitung, die durch die Verbinderbohrung (150) aufgenommen wird, wobei bestimmt wird, dass der medizinische Leitungstyp eine einer integriert bipolaren Leitung oder einer echt bipolaren Leitung ist;
Auswählen wenigstens einer Betriebsparametereinstellung basierend auf dem bestimmten medizinischen Leitungstyp;
Verarbeiten eines elektrischen Herzsignals gemäß der wenigstens einen Betriebsparametereinstellung zum Bestimmen eines Bedarfs an einer elektrischen Stimulationstherapie; und
Bereitstellen der elektrischen Stimulationstherapie als Reaktion auf die Bestimmung des Bedarfs an der elektrischen Stimulationstherapie.

## Revendications

1. Dispositif médical (14) comprenant :
un alésage de connecteur (150) configuré pour recevoir une partie proximale d'un conducteur médical (60), l'alésage de connecteur (150) comprenant au moins un premier contact électrique (160) et un second contact électrique (162) ;
un circuit de mesure d'impédance (90) configuré pour obtenir une mesure d'impédance entre une première borne d'électrode correspondant au premier contact électrique (160) de l'alésage de connecteur (150) et une seconde borne d'électrode correspondant au second contact électrique (162) de l'alésage de connecteur (150) ; et
un circuit de commande (80) configuré pour :
déterminer un type de conducteur médical du conducteur médical (60) reçu par l'alésage de connecteur (150) sur la base de la mesure d'impédance, le type de conducteur médical étant déterminé comme étant un conducteur bipolaire intégré ou un conducteur bipolaire vrai ; et
sélectionner au moins un réglage de paramètre de fonctionnement en fonction du type de conducteur médical déterminé ; et
un circuit de traitement de signal (86) configuré pour recevoir un signal électrique cardiaque et traiter le signal électrique cardiaque en fonction de l'au moins un réglage de paramètre de fonctionnement, afin de déterminer le besoin d'une thérapie de stimulation électrique ; et
un circuit d'administration de thérapie (84) configuré pour administrer la thérapie de stimulation électrique en réponse à la détermination par le circuit de traitement de signal (86) du besoin de la thérapie de stimulation électrique.

2. Dispositif médical (14) selon la revendication 1,
le circuit de traitement de signal (86) comprenant un filtre coupe-bande ; et
le circuit de commande (80) étant en outre configuré pour sélectionner l'au moins un réglage de paramètre de fonctionnement en activant le filtre coupe-bande en réponse à la détermination que le type de conducteur médical est le conducteur bipolaire intégré.

3. Dispositif médical (14) selon l'une quelconque des revendications 1 et 2,
le circuit de traitement de signal (86) étant configuré pour détecter la surdétection d'événement cardiaque ; et le circuit de commande (80) étant en outre configuré pour sélectionner l'au moins un réglage de paramètre de fonctionnement par l'une parmi :
l'activation de la surdétection d'événement cardiaque par le circuit de traitement de signal (86) en réponse à la détermination que le type de conducteur médical est le conducteur bipolaire intégré ; ou
la désactivation de la surdétection d'événement cardiaque par le circuit de traitement de signal (86) en réponse à la détermination que le type de conducteur médical est le conducteur bipolaire vrai.

4. Dispositif médical (14) selon la revendication 3, le circuit de traitement de signal (86) étant en outre configuré pour détecter la surdétection d'événement cardiaque en détectant la surdétection d'onde P.

5. Dispositif médical (14) selon l'une quelconque des revendications 1 et 4,
le circuit de traitement de signal (86) étant configuré pour détecter une surdétection d'événement cardiaque par :
la détection d'une pluralité de signaux d'événement cardiaque à partir du signal électrique cardiaque ;
la détermination d'au moins une caractéristique de signal à partir de chacun de la pluralité de signaux d'événement cardiaque ; et
la détermination d'un motif alternatif des caractéristiques de signal déterminées sur la base d'au moins un seuil appliqué aux caractéristiques de signal déterminées ; et
le circuit de commande (80) étant en outre configuré pour sélectionner l'au moins un réglage de paramètre de fonctionnement par l'une parmi :
la sélection d'une première valeur du seuil en réponse à la détermination que le type de conducteur médical est le conducteur bipolaire intégré ; ou
la sélection d'une seconde valeur du seuil différente de la première valeur en réponse à la détermination que le type de conducteur médical est le conducteur bipolaire vrai.

6. Dispositif médical (14) selon l'une quelconque des revendications 1 et 5,
le circuit de traitement de signal (86) étant configuré pour détecter une surdétection d'événement cardiaque par :
la détection d'une pluralité de signaux d'événement cardiaque à partir du signal électrique cardiaque ;
la détermination d'au moins une caractéristique de signal à partir de chacun de la pluralité de signaux d'événement cardiaque ; et
la détermination d'un motif alternatif des caractéristiques de signal déterminées sur la base d'au moins un seuil appliqué aux caractéristiques de signal déterminées ; et
le circuit de commande (80) étant en outre configuré pour sélectionner l'au moins un réglage de paramètre de fonctionnement par l'une parmi :
la sélection d'une première caractéristique de signal à déterminer par le circuit de traitement de signal (86) parmi chacun de la pluralité de signaux d'événement cardiaque en réponse à la détermination que le type de conducteur médical est le conducteur bipolaire intégré ; ou
la sélection d'une seconde caractéristique de signal à déterminer par le circuit de traitement de signal (86) à partir de chacun de la pluralité de signaux d'événement cardiaque en réponse à la détermination que le type de conducteur médical est le conducteur bipolaire vrai, la seconde caractéristique de signal étant différente de la première caractéristique de signal.

7. Dispositif médical (14) selon l'une quelconque des revendications 1 et 6,
le circuit de traitement de signal (86) étant en outre configuré pour détecter des signaux de bruit non cardiaque dans le signal électrique cardiaque ; et
le circuit de commande (80) étant en outre configuré pour sélectionner l'au moins un réglage de paramètre de fonctionnement en permettant de détecter des signaux de bruit non cardiaque dans le signal électrique cardiaque par le circuit de traitement de signal (86) en réponse à la détermination que le type de conducteur médical est le conducteur bipolaire intégré.

8. Dispositif médical (14) selon l'une quelconque des revendications 1 et 7,
le circuit de traitement de signal (86) étant en outre configuré pour :
déterminer un score de concordance morphologique entre un modèle morphologique préalablement établi correspondant à un signal d'événement cardiaque et un signal d'événement inconnu dans le signal électrique cardiaque ; et
comparer le score de concordance morphologique à un seuil de concordance morphologique pour déterminer si le signal inconnu est le signal d'événement cardiaque ; et
le circuit de commande (80) étant en outre configuré pour sélectionner l'au moins un réglage de paramètre de fonctionnement par l'une parmi :
la sélection d'une première valeur du seuil de concordance morphologique en réponse à la détermination que le type de conducteur médical est le conducteur bipolaire intégré ; ou
la sélection d'une seconde valeur du seuil de concordance morphologique différente de la première valeur en réponse à la détermination que le type de conducteur médical est le conducteur bipolaire vrai.

9. Dispositif médical (14) selon l'une quelconque des revendications 1 et 8,
le circuit de traitement de signal (86) étant en outre configuré pour :
déterminer un score de concordance morphologique entre un modèle morphologique préalablement établi correspondant à un signal d'événement cardiaque et un signal d'événement inconnu dans le signal électrique cardiaque ; et
comparer le score de concordance morphologique à un seuil de concordance morphologique pour déterminer si le signal d'événement inconnu est le signal d'événement cardiaque ; et
le circuit de commande (80) étant en outre configuré pour sélectionner l'au moins un réglage de paramètre de fonctionnement par :
la détermination que le type de conducteur médical est différent d'un type de conducteur médical déterminé précédemment ; et
le rétablissement du modèle de morphologie à partir du signal électrique cardiaque en réponse à la détermination que le type de conducteur médical est différent du type de conducteur médical précédemment déterminé.

10. Dispositif médical (14) selon l'une quelconque des revendications 1 à 9, comprenant en outre une mémoire,
le circuit de mesure d'impédance (90) étant en outre configuré pour déterminer une mesure d'impédance de conducteur bipolaire ; et
le circuit de commande (80) étant en outre configuré pour :
sélectionner l'au moins un réglage de paramètre de fonctionnement par l'une parmi :
la sélection d'une première plage d'impédance de conducteur bipolaire normale en réponse à la détermination que le type de conducteur médical est le conducteur bipolaire intégré ; ou
la sélection d'une seconde plage de la plage d'impédance normale du conducteur bipolaire en réponse à la détermination que le type de conducteur médical est le conducteur bipolaire vrai, la seconde plage étant différente de la première plage ;
déterminer que la mesure d'impédance du conducteur bipolaire est en dehors de la plage normale d'impédance du conducteur bipolaire ; et
générer une sortie en réponse à la détermination que la mesure d'impédance du conducteur bipolaire est en dehors de la plage normale d'impédance du conducteur bipolaire ; et
la mémoire étant configurée pour stocker la mesure d'impédance du conducteur bipolaire avec la sortie générée par le circuit de commande (80).

11. Dispositif médical (14) selon l'une quelconque des revendications 1 à 10, comprenant en outre un circuit de télémétrie configuré pour transmettre des signaux de données :
le circuit de commande (80) étant en outre configuré pour définir au moins un paramètre de rapport sur la base du type de piste déterminé ; et
le circuit de télémesure étant configuré pour transmettre le paramètre de rapport à un autre dispositif médical (14) destiné à être utilisé pour générer un affichage d'une interface utilisateur graphique.

12. Dispositif médical (14) selon l'une quelconque des revendications 1 à 11, le circuit de commande (80) étant en outre configuré pour déterminer le type de conducteur médical par :
la comparaison de la mesure d'impédance à une impédance seuil ;
en réponse à la mesure d'impédance inférieure à l'impédance de seuil, la détermination que le type de conducteur médical est le conducteur bipolaire intégré ayant une première électrode de bobine allongée du conducteur bipolaire intégré couplée électriquement à la première borne d'électrode et couplée électriquement à la seconde borne d'électrode ; ou
en réponse à la mesure d'impédance supérieure à l'impédance de seuil, la détermination que le type de conducteur médical est le conducteur bipolaire vrai ayant une seconde électrode de bobine allongée du conducteur bipolaire vrai couplée électriquement à la première borne d'électrode et une électrode annulaire du conducteur bipolaire vrai couplée électriquement à la seconde borne d'électrode.

13. Support non transitoire lisible par ordinateur stockant un ensemble d'instructions qui, lorsqu'elles sont exécutées par un circuit de commande (80) d'un dispositif médical (14), amènent le dispositif médical (14) à :
obtenir une mesure d'impédance entre une première borne d'électrode correspondant à un premier contact électrique (160) d'un alésage de connecteur (150) du dispositif médical (14) et une seconde borne d'électrode correspondant à un second contact électrique (162) de l'alésage de connecteur (150), l'alésage de connecteur (150) étant configuré pour recevoir une partie proximale d'un conducteur médical (60) ;
sur la base de la mesure d'impédance, déterminer un type de conducteur médical du conducteur médical reçu par l'alésage de connecteur (150), le type de conducteur médical étant déterminé comme étant un conducteur bipolaire intégré ou un conducteur bipolaire vrai ;
sélectionner au moins un réglage de paramètre de fonctionnement en fonction du type de conducteur médical déterminé ;
traiter un signal électrique cardiaque en fonction de l'au moins un réglage de paramètre de fonctionnement pour déterminer le besoin d'une thérapie de stimulation électrique ; et
administrer la thérapie de stimulation électrique en réponse à la détermination du besoin de la thérapie de stimulation électrique.
